# EUROPEAN PATENT APPLICATION

(11) **EP 1 667 060 A1**
(43) Date of publication of application: **07.06.2006**
(21) Application number: 04773185.6
(22) Date of filing: 16.09.2004
(51) Int. Cl.: G06Q 10/00

(54) **MEDICINE RESEARCH INFORMATION COLLECTION SYSTEM**

(30) Priority: 17.09.2003 JP 2003325200
(71) Applicant: Astellas Pharma Inc., Tokyo 103-8411 (JP)
(72) Inventor: MORIYAMA, K., Astellas Pharma Inc, Tokyo 1748612 (JP)
(74) Representative: Grünecker, Kinkeldey, Stockmair & Schwanhäusser Anwaltssozietät
(86) International application number: PCT/JP2004/013527
(87) International publication number: WO 2005/029380

(57) **Abstract**

[Object] An object of the present invention is to provide a medicine research information collection form capable of organizing contents of a plurality of collected medicine research information collection forms into a database with good efficiency.

[Solving Means] The present invention relates to a medicine research information collection form 20 for medicine research requiring statistical processing for verifying validity of research results obtained from electronic input information generated based on handwritten information on a medicine research case. The medicine research information collection form 20 of the present invention is a sheet of paper having a printed position encoding pattern P for deciding a coordinate position of an electronic pen 10 on the paper. Furthermore, the sheet of paper has a printed item for handwriting or selecting information on medicine research such as a clinical trial.

## Description

### Technical Field

The present invention relates to a medicine research information collection system, a medicine research information collection form, a medicine research information collection method, a medicine research information collection device, and a medicine research information collection program, more particularly to a medicine research information collection system, a medicine research information collection form, a medicine research information collection method, a medicine research information collection device, and a medicine research information collection program for collecting medicine research information in a clinical test and the like.

### Background Art

Heretofore, a clinical trial has been performed in a final stage of a new drug developing process. The clinical trial is performed in accordance with a clinical trial performing plan made by a clinical trial client such as a pharmaceutical company based on the pharmaceutical law (GCP related act), and a case report form (hereinafter referred to as "CRF") prepared by the clinical trial client is used in the clinical trial. The CRF is a report form for recording, with respect to individual subjects, detailed information on age, gender, medicine for use, dose of the medicine, the number of administration times, effects produced by the medicine, clinical observation, results of a clinical inspection, and a clinical trial course such as side effects. This CRF has formats and input columns which differ with the clinical trials, and is prepared for each of the subjects as clinical trial objects.

Moreover, the CRF is filled in by the following procedure in a clinical site during the clinical trial. That is, the clinical trial client distributes the CRFs to medical institutions in which the clinical trials are performed. A doctor in charge of the clinical trial in the medical institution writes, in predetermined columns of the CRF, location of the medical institution, facility name, department name, initials of the subject as the clinical trial object, carte number and the like. After actually performing the clinical test, the doctor in charge of the clinical trial or a nurse transfers, to the predetermined columns of the CRF, information during the use of the investigational drug, states of the subject before and after the use of the investigational drug, clinical inspection results of blood, urine and the like described in original data such as the carte and an inspection form. Furthermore, as to items which are not described in the original data and for which medical judgments are required, safety evaluation and effect judgment of the investigational drug and the like, the doctor in charge of the clinical trial directly fills in the CRF. Subsequently, after completion of the writing of the information on the clinical trial into the CRF, the doctor in charge of the clinical trial signs a signature column of the CRF, thereby completing the fill-in of the CRF in the clinical site.

The CRFs which have been filled in as described above are collected by the clinical trial client, and contents of the collected CRFs are input as electronic data into a database by a special puncher. In this case, in order to prevent mistakes in inputting the information into the database, a double input operation is performed to input the information into the same CRF twice. The clinical trial client checks the contents input into the database for fill-in leak, fill-in mistake, cacography/lipography, protocol violation and the like. If there is a problem, the doctor in charge of the clinical trial, who has filled in, is inquired about the problem.

Moreover, if there is a mistake or the like in the contents of the CRF, the doctor in charge of the clinical trial is requested to correct it. In a case where the contents of the CRF are corrected, the clinical trial client inputs the contents into the database again, and prepares a correction history. Furthermore, in a case where it can be confirmed that there is not any more problem in the contents of the CRF input into the database, the data input into the database is fixed. The data of the database fixed in this manner is statistically processed, and utilized in the data for approval of manufacturing of the investigational drug.

Here, in a case where the contents of the collected CRFs are input into the database as described above, when the double input operation is performed with respect to the same CRF, or a reading operation for reading and comparing the electronic data and the CRF to search for any mistake is performed in order to prevent the input mistakes, there is a problem that much time and energy are spent. Furthermore, in a case where the CRF is a booklet, all of items described in the booklet cannot be input into the database unless they are filled in, signed, or sealed by a doctor in charge of the clinical trial such as a doctor responsible for the clinical trial or a doctor in charge of part of the clinical trial. For the purpose of improving this problem, there is provided one or a plurality of forms of visit type (a sheet is cut off so that the sheet can be signed or sealed every time the subject visits the doctor), but it has been difficult to efficiently collect and input the medicine research information in any case.

Therefore, in view of such problem, the following system utilizing a computer is developed (see, e.g., Japanese Patent Application Laid-Open No. 2003-223514). That is, in this conventional example, an electronic CRF sheet having the same format as that of paper CRF which has heretofore been used is displayed in a display device of a computer system, and the doctor directly inputs the data into this displayed electronic CRF sheet. According to this system, since it is not necessary to transfer the clinical trial data from the paper CRF to the electronic CRF sheet in the computer, the double input operation becomes unnecessary. When abnormal value judgment software or the like is incorporated into the computer, an abnormal value can be detected in an input stage. Therefore, there is a merit that inquiries to the doctor can be reduced. There is also a merit that it is possible to grasp clinical trial progress situations because the data can be collected in real time.

### Disclosure of the Invention

### Problems to be Solved by the Invention

However, in the above-described conventional example, exclusive-use software for inputting and processing clinical trial data needs to be installed beforehand in a computer installed in a clinical site. In a case where the computer installed in the clinical site cannot be utilized owing to various factors, it is necessary for a clinical trial requesting maker to lend an exclusive-use computer to a hospital which is a clinical site. Therefore, in a case where the clinical trials are requested from a plurality of pharmaceutical companies in one clinical site, a plurality of computers are installed in the clinical site. This disadvantageously brings the clinical site into a troublesome state.

Furthermore, since the doctor in charge of the clinical trial needs to be trained in screen input, and a way to use the software differs with a clinical trial requesting maker, there is a disadvantage that burdens on the doctor in charge of the clinical trial are large. Therefore, eventually, the nurse or a development staff member in charge of the clinical trial needs to bring the computer and perform the input operation in lieu of the doctor in charge of the clinical trial. In such situation, it is more efficient to transfer the contents of the carte or the inspection form to the paper CRF so that the special puncher inputs the contents described in this CRF into the computer as before, as compared with a case where the computer system is used as in the above-described conventional example.

Moreover, according to the above-described conventional method, the information input into the database is once printed out. Instead of assuring that there is not any discrepancy in the database input contents, for the sake of convenience, printed-out paper is signed or sealed by the doctor in charge of the clinical trial or the like to form the original data to be submitted to a relevant government ministry or agency. However, the statistical processing is performed based on the information input into the database, and there is a problem that the processing has to depend on irregular processing while both of electronic information that has to be said to be an electronic original and a paper original exist in a mixed manner. Various problems described above are common to various research information such as information of post-marketing research, patient registration, and questionnaire, and it has been demanded that there be provided a research information collection form and a research information collection method which can solve these problems.

The present invention has been developed in view of the above-described situations, and an object thereof is to provide a medicine research information collection system, a medicine research information collection form, a medicine research information collection method, a medicine research information collection device, and a medicine research information collection program, capable of efficiently organizing contents of a plurality of medicine research information collection forms into a database.

Moreover, another object of the present invention is to provide a medicine research information collection system, a medicine research information collection form, a medicine research information collection method, a medicine research information collection device, and a medicine research information collection program, capable of correctly organizing contents of medicine research information collection forms filled in by a doctor in charge of a clinical trial and the like into a database.

### Means for Solving the Problems

To solve the above-described problems, the present invention provides a medicine research information collection system for collecting medicine research information, comprising a medicine research information collection form which has coordinate position deciding means for deciding a coordinate position on an input face and in which an item capable of handwriting or selecting at least the medicine research information is displayed in a field of the input face, a handwriting device which is constituted to be capable of handwriting the medicine research information in the input face and which computerizes the handwritten medicine research information based on the coordinate position deciding means to transmit the information, and a database device including a database which receives the medicine research information transmitted from the handwriting device and which accumulates the received medicine research information.

According to the present invention, since the contents written in the medicine research information collection form can be directly organized into the database, it is possible to save labor for inputting, into a computer, the contents written in the research information collection form by a person in charge of research and the like, and it is also possible to prevent input mistakes from being generated in a database organizing stage.

Here, in the medicine research information collection system, more preferably the input face of the medicine research information collection form is provided with a start check box to be checked when the medicine research information starts to be written using the handwriting device, and an end check box to be checked when the writing of the medicine research information is completed. The handwriting device transmits an input start signal to the database device in response to the checking of the start check box, outputs an input end signal to the database device in response to the checking of the end check box, computerizes the medicine research information written in the input face of the medicine research information collection form from a time when the start check box is checked until the end check box is checked, and transmits the information to the database device. The database device starts the input of the medicine research information transmitted from the handwriting device based on the input start signal transmitted from the handwriting device, and completes the input of the medicine research information transmitted from the handwriting device based on the input end signal transmitted from the handwriting device. According to such constitution, a region of data of the medicine research information written with the handwriting device can be clarified, and it is accordingly possible to securely store the data.

Moreover, in the medicine research information collection system, more preferably, the database device comprises user identification information storage means for storing beforehand user identification information which identifies a user, user identification information input means for the user to input the user's user identification information, user identification information judgment means for judging whether or not the user identification information input by the user identification information input means agrees with that stored beforehand in the user identification information storage means, and permission means for permitting the user to use the database device in a case where the user identification information input by the user identification information input means agrees with that stored beforehand in the user identification information storage means. According to such constitution, since a specific user only is permitted to use the database device, it is possible to prevent leak of the medicine research information.

Furthermore, in the medicine research information collection system, more preferably, the handwriting device is constituted to transmit identification information for identifying each handwriting device. The database device comprises handwriting device identification information storage means for storing beforehand the identification information of each handwriting device, identification information receiving means for receiving the identification information transmitted from the handwriting device, and handwriting device identification means for judging whether or not the user identification information received by the identification information receiving means agrees with the identification information stored beforehand in the handwriting device identification information storage means to thereby identify the handwriting device which has transmitted the medicine research information. According to such constitution, by means of the identification information of the handwriting device, it is possible to grasp the individual handwriting device, a manager, a managing division, a clinical trial management number and the like of the handwriting device, and it is possible to specify a facility in which the handwriting device is used.

In addition, in the medicine research information collection system, more preferably, the database device comprises correction judgment means for judging whether or not the medicine research information transmitted from the handwriting device corrects the medicine research information already stored in the database, and correction information storage means for storing the medicine research information transmitted from the handwriting device as corrected medicine research information in the database in a case where the correction judgment means judges that the medicine research information transmitted from the handwriting device corrects the medicine research information already stored in the database. According to such constitution, in addition to the medicine research information already stored in the database, the corrected medicine research information can be stored while distinguished from the already stored medicine research information.

Moreover, in the medicine research information collection system, more preferably, the database device comprises statistical processing means for statistically processing the medicine research information transmitted from the handwriting device. According to such constitution, it is possible to statistically process the medicine research information accumulated in the database.

Furthermore, in the medicine research information collection system, more preferably, the database device comprises a primary database device which primarily stores the medicine research information transmitted from the handwriting device, and a fixed database device which stores the medicine research information stored in the primary database device as fixed information. According to such constitution, it is possible to use the primary database while the medicine research information is collected, and use the fixed database device when the collecting of the medicine research information is completed. Since the medicine research information is transferred to the fixed database device after completing the collecting of the medicine research information in this manner, the information can be prevented from being leaked or tampered.

Additionally, to solve the above-described problem, a medicine research information collection form of the present invention has coordinate position deciding means for deciding a coordinate position on an input face, and displays an item capable of handwriting or selecting at least the medicine research information in a field of the input face.

According to the present invention, the medicine research information collection form has the coordinate position deciding means for deciding the coordinate position on the input face, and there are displayed the item for handwriting or selecting at least the medicine research information in the field of the input face,. Therefore, contents handwritten in this research information collection form can be computerized, using, for example, the handwriting device capable of computerizing the handwritten contents. Furthermore, electronic information can be accumulated to form the database more easily. This can save a trouble of inputting the contents written in the research information collection form into the computer by a person in charge of research or the like, and it is possible to prevent input mistakes or the like in a database organizing stage.

Here, the input face of the medicine research information collection form is more typically paper or a screen of a display.

To be more specific, the coordinate position deciding means of the medicine research information collection form is means for deciding the coordinate position of the handwriting device in the input face during the handwriting on the input face by use of the handwriting device. When the coordinate position deciding means is disposed in this manner, it is possible to computerize the handwritten medicine research information based on the coordinate position deciding means. It is to be noted that the coordinate position deciding means is preferably a raster point optically readable by an electronic pen as the handwriting device because it is possible to specify a coordinate of the handwriting device more correctly.

Furthermore, the item to be handwritten or selected in the medicine research information collection form is more typically at least a particular concerning a facility in which medicine research is performed, a particular concerning a doctor who performs the medicine research, initials of a subject or a patient, or a particular concerning contents of the medicine research, or a combination of them.

In addition, the item to be handwritten or selected in the medicine research information collection form is more typically at least a title concerning the medicine research, a particular concerning the medicine to be subjected to the medicine research, the particular concerning the facility in which the medicine research is performed, the particular concerning the doctor who performs the medicine research, the initials of the subject or the patient, or the particular concerning the contents of the medicine research, or a combination of them.

Moreover, the contents of the medicine research in the medicine research information collection form are more typically particulars concerning the subject or the patient, particulars concerning inspection of the subject or the patient, which is performed during medicine administration, particulars concerning effects of the medicine administration to the subject or the patient, or particulars concerning influences of the medicine administration on the subject or the patient, or a combination of them.

Furthermore, to solve the above-described problem, the present invention provides a medicine research information collection method for collecting medicine research information, the method comprising an input step of handwriting or selecting the medicine research information on an input face of a medicine research information collection form by use of a handwriting device, a computerizing step of computerizing the medicine research information handwritten on the input face of the medicine research information collection form in the handwriting device, a transmitting step of transmitting the medicine research information computerized in the computerizing step to a database device, a receiving step of receiving the medicine research information transmitted from the handwriting device in the database device, and an accumulating step of accumulating the medicine research information received in the receiving step in a database of the database device.

According to the present invention, since contents written in the medicine research information collection form can be directly organized into a database by the above-described method, it is possible to save a trouble of inputting the contents written in the research information collection form into a computer by a person in charge of the research or the like, and it is possible to prevent input mistakes or the like in a database organizing stage.

Moreover, in the medicine research information collection method, more specifically, when the information is handwritten on the input face of the medicine research information collection form by use of the handwriting device, a coordinate position of the handwriting device in the input face is decided based on coordinate position deciding means disposed on the input face of the medicine research information collection form. In this case, it is possible to computerize the handwritten medicine research information based on the coordinate position deciding means. It is to be noted that when the handwriting device constituted of an electronic pen capable of optically reading a raster point as the coordinate position deciding means is used, it is preferably possible to specify a coordinate of the handwriting device more correctly.

Furthermore, to solve the above-described problems, the present invention provides a medicine research information collection device for collecting medicine research information, comprising receiving means for receiving the medicine research information transmitted from a handwriting device capable of computerizing contents handwritten in a medicine research information collection form, and a database which accumulates the medicine research information received by the receiving means.

According to the present invention, since the contents written in the medicine research information collection form can be directly organized into a database, it is possible to save a trouble of inputting the contents written in the research information collection form into a computer by a person in charge of the research or the like, and it is possible to prevent input mistakes or the like in a database organizing stage.

Moreover, to solve the above-described problems, the present invention allows a computer disposed in a medicine research information collection device for collecting medicine research information to execute a medicine research information receiving step of receiving the medicine research information transmitted from a handwriting device capable of computerizing contents handwritten in a medicine research information collection form, and a medicine research information accumulating step of accumulating the medicine research information received by the receiving step in a database.

According to the present invention, since the contents written in the medicine research information collection form can be directly organized into a database, it is possible to save a trouble of inputting the contents written in the research information collection form into the computer by a person in charge of the research or the like, and it is possible to prevent input mistakes or the like in a database organizing stage.

Here, more preferably, the medicine research information collection program allows the computer disposed in the medicine research information collection device to execute an input start step of starting input of the medicine research information transmitted from the handwriting device based on an input start signal transmitted from the handwriting device, when a start check box disposed in an input face of the medicine research information collection form is checked, and an input end step of completing the input of the medicine research information transmitted from the handwriting device based on an input end signal transmitted from the handwriting device, when an end check box disposed in the input face of the medicine research information collection form is checked. In this case, a region of data of the medicine research information written with the handwriting device can be clarified, and it is accordingly possible to securely store the data.

Moreover, the above-described medicine research information collection program more preferably allows the computer disposed in the medicine research information collection device to execute a user identification information storing step of storing beforehand user identification information which identifies a user, user identification information judgment means of judging whether or not the user identification information input by user identification information input means agrees with that stored beforehand in the user identification information storing step, and a permitting step of permitting the user to use the medicine research information collection device in a case where it is judged that the user identification information input by the user identification information input means agrees with that stored beforehand in user identification information means. In this case, since a specific user only can be permitted to use the database device, leak of the medicine research information can be prevented.

Furthermore, the above-described medicine research information collection program more preferably allows the computer disposed in the medicine research information collection device to execute a handwriting device identification information storing step of storing beforehand identification information of each handwriting device, an identification information receiving step of receiving the identification information transmitted from the handwriting device, and a handwriting device identifying step of judging whether or not the user identification information received in the identification information receiving step agrees with the identification information stored beforehand in the handwriting device identification information storing step to thereby identify the handwriting device which has transmitted the medicine research information. In this case, by means of the identification information of the handwriting device, it is possible to grasp the individual handwriting device, a manager, a managing division, a clinical trial management number and the like of the handwriting device, and it is possible to specify a facility in which the handwriting device is used.

In addition, the above-described medicine research information collection program more preferably allows the computer disposed in the medicine research information collection device to execute a correction judging step of judging whether or not the medicine research information transmitted from the handwriting device corrects the medicine research information already stored in a database, and a correction information storing step of storing the medicine research information transmitted from the handwriting device as corrected medicine research information in the database in a case where correction judgment means judges that the medicine research information transmitted from the handwriting device corrects the medicine research information already stored in the database. In this case, in addition to the medicine research information already stored in the database, the corrected medicine research information can be stored while distinguished from the already stored medicine research information.

Moreover, the above-described medicine research information collection program more preferably allows the computer disposed in the medicine research information collection device to execute a statistical processing step of statistically processing the medicine research information transmitted from the handwriting device. In this case, it is possible to statistically process the medicine research information accumulated in the database.

### Advantages of the Invention

As described above, according to the present invention, since it is possible to constitute the contents written in the medicine research information collection form directly into the database, it is possible to save a trouble of inputting the contents written in the research information collection form into the computer by a person in charge of research or the like, and it is possible to constitute the contents of a plurality of collected research information collection forms into the database with a good efficiency.

Moreover, according to the research information collection method of the present invention, as described above, since it is possible to constitute the contents written in the research information collection form directly into the database, input mistakes or the like are prevented in a database organizing stage, and it is possible to correctly constitute the contents written in the research information collection form into the database.

### Best Mode for Carrying out the Invention

One embodiment of the present invention will be described hereinafter with reference to the drawings. It is to be noted that, needless to say, members, arrangements and the like described below do not restrict the present invention, and can be variously altered in accordance with the scope of the present invention.

### (Whole constitution of medicine research information collection system)

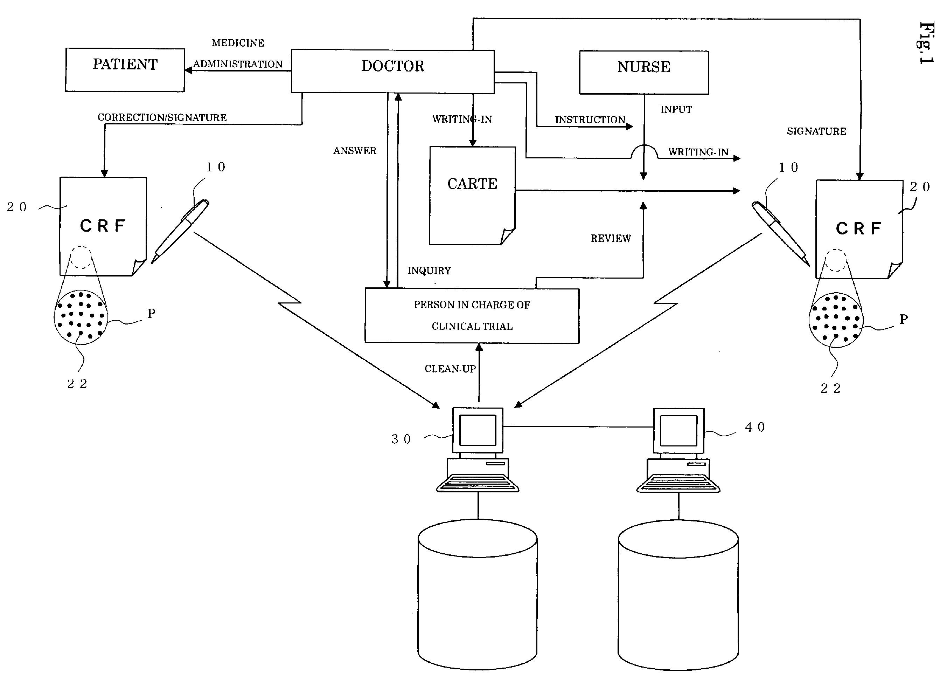

First, a constitution of a medicine research information collection system will be described in one embodiment of the present invention with reference to FIGS. 1 to 16. A medicine research information collection system S of the present embodiment is preferably used in, for example, organizing, into a database, contents of a plurality of case report forms (hereinafter referred to as CRF) described by a doctor in a clinical trial or the like. This medicine research information collection system S is constituted of an electronic pen 10, a medicine research information collection form 20, a primary database device 30, and a fixed database device 40.

### (Constitution of electronic pen)

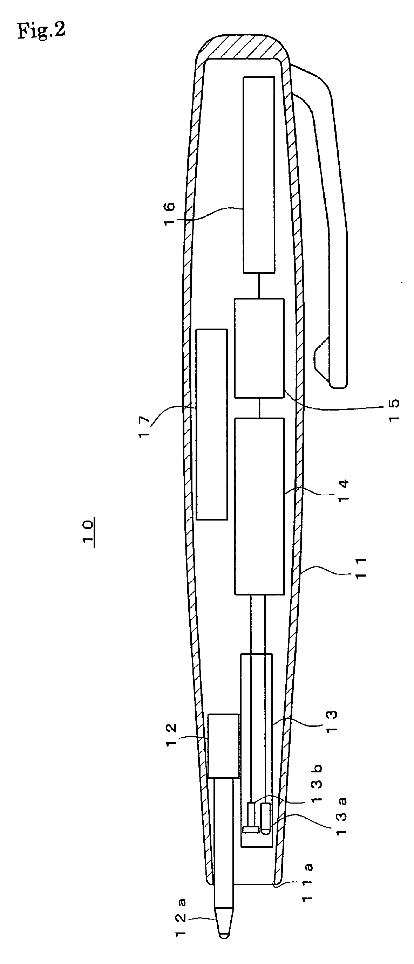

The electronic pen 10 has a function of writing characters or the like in the medicine research information collection form 20, and a function of reading position encoding pattern P printed on paper to thereby record, as electronic input information, a movement track of a pen tip during the writing of the characters, and preferably transmitting this electronic input information to a receiving terminal connected via Bluetooth (registered trademark) interface. As shown in FIG. 2, the electronic pen 10 has a case 11, an ink unit 12, a camera 13, a processor unit 14, a memory 15, a transmission unit 16, and a battery 17.

The case 11 is constituted into a shape substantially similar to that of an outer cylindrical portion of a fountain pen, a ball point pen or the like for general use, and has an opening 11a in a tip portion thereof. The ink unit 12 is fixed to an inner tip portion of the case 11. This ink unit 12 is constituted to discharge ink from a tip portion 12a thereof, and the tip portion 12a of the ink unit 12 protrudes outwardly from the opening 11a in an axial direction of the pen. Moreover, according to this constitution, it is possible to write characters and the like on paper by use of the electronic pen 10 in the same manner as in the fountain pen, the ball point pen or the like for general use.

The camera 13 is disposed close to the ink unit 12 in the inner tip portion of the case 11. This camera 13 is provided with a light emitting diode 13a and a photosensitive area sensor 13b such as a CCD or CMOS sensor which records a two-dimensional image. The light emitting diode 13a is constituted so as to emit an infrared ray from its tip portion to the outside of the pen in the axial direction. The photosensitive area sensor 13b is constituted so as to detect light reflected by the paper from the infrared ray emitted from the light emitting diode 13a to the paper. It is to be noted that if necessary, a mirror, a lens or the like may be disposed between the camera 13 and the opening 11a of the case 11.

The processor unit 14 has a function of controlling the camera 13, and is provided with program for deciding a position of a raster point 22 printed on the medicine research information collection form 20 in an image recorded by the photosensitive area sensor 13b, and coding the position to specify a position of a pen tip from an accordingly obtained code. The processor unit 14 is constituted so as to continuously input data from the photosensitive area sensor 13b, accordingly analyze a movement track of the pen tip, and store the analyzed data in the memory 15.

It is to be noted that the processor unit 14 may be constituted so as to transform a direction or a speed of movement of the pen tip into a data, and stores this data in the memory 15 together with coordinate data concerning the movement track. Moreover, the processor unit 14 in the present embodiment has a function of controlling the transmission unit 16, and is constituted so as to transmit the data stored in the memory 15 to the receiving terminal (computer 31 in the present embodiment). The transmission unit 16 radio-transmits the data stored in the memory 15 to the receiving terminal connected via Bluetooth (registered trademark) interface, and the battery 17 supplies power to the camera 13, the processor unit 14, the memory 15, and the transmission unit 16.

Moreover, the electronic pen 10 of the present embodiment is constituted to start, when a cap (not shown) is removed from the case 11 or a switch (not shown) is turned on. The electronic pen 10 can transmit the data stored in the memory 15 to the primary database device 30. In this case, the data transmitted from the electronic pen 10 includes a pen ID signal indicating ID of the pen. It is to be noted that, for example, Anoto pen manufactured by Anoto Group AB is usable as a pen having a function of writing the characters and the like in the medicine research information collection form 20, and a function of reading the position encoding pattern P printed on the paper to thereby record as the electronic input information a movement track of the pen tip during the writing of the characters, and transmitting the electronic input information to the receiving terminal connected via Bluetooth (registered trademark) interface as described above.

### (Constitution of medicine research information collection form)

The medicine research information collection form 20 is distributed to a medical institution in which the clinical trial (or post-marketing research, questionnaire, etc.) is performed by a clinical trial client (research client in post-marketing research) as a pharmaceutical company. In the form, a doctor, a nurse and the like write research information, especially research information on the medicine as requested by the pharmaceutical company in the clinical site. It is assumed that in the present embodiment, as one example of the medicine research information collection form 20, there is used a case report form, a use result research form, a use result research registration form, or a patient registration form. It is to be noted that here the patient registration form has been described, the form of the present invention is not limited to a booklet type form, and the form may be a portion of information of a part of the booklet type as in so-called visit type.

The use result research form is constituted of a cover sheet and a research content page. Descriptions of print items and printing style of the use result research form, notes and description knack for the doctor in charge of the clinical trial or the like to fill in the form and the like variously differ with the pharmaceutical company as the clinical trial client or an adaptation disease of the investigational drug as an object. For example, in the embodiment of a clinical case report form of one of diabetes mellitus therapeutic drugs, the cover sheet is provided with a case number column, a research object name column, a sending notice number column, a title column, a facility name and location column, a facility name column, an in-facility department name column, a patient initial column, a carte number column, a doctor name and authentication column, and a fill-in date column. In the title column of the cover sheet, name, abbreviation or the like of a used medicine is preferably written. The research content page is provided with a patient background column, a therapy column, a clinical course column, an effect judgment column, a clinical inspection column, a clinical inspection value abnormal fluctuation column, and an adverse event column.

The patient background column is provided with columns for describing birth date, occupation, gender, pregnancy, height, weight, reason for use, segment, disease contraction period, family history, complication, medical history, hypersensitiveness identity, previous therapeutic drug and the like. The therapy column is provided with columns for describing present drug dose, the number of medicine administration times, medicine administration period, presence of discontinuance/dropout, combined medicine name, combined medicine administration route, combined medicine dose per day, combined medicine administration period, and reason for use of combined medicine. The clinical course column is provided with columns for describing an inspection item, a pre-medicine-administration value of each inspection item, an inspected value a certain period after start of the medicine administration, and an inspected value during the discontinuance/dropout of the medicine administration.

Moreover, the effect judgment column is provided with columns for writing general judgment, grounds for the judgment, and comment. The clinical inspection column is provided with columns for describing the presence of implementation, an inspection item, a pre-medicine-administration value of each inspection item, and an inspected value of each inspection item during/after the medicine administration. The clinical inspection value abnormal fluctuation column is provided with columns for describing an abnormal fluctuation judged to be clinically significant, and comment on the clinical inspection value abnormal fluctuation. The adverse event column is provided with columns for describing the presence of an adverse event, a type of the adverse event, seriousness, treatment (the present medicine administration and/or therapy), a causal relation with respect to the present medicine, another suspected combined medicine/combined therapy and the like.

As one example of the medicine research information collection form 20, a use result research registration form as a participation registration form in the post-marketing research of a marketed oral contraceptive is constituted of a cover sheet and a research content page. This registration form also variously differs with the pharmaceutical company which is a post-marketing research client. In this example, the cover sheet is provided with a registration number column, a title column, a location column, a facility name column. a department column, a doctor name and authentication column, a carte number column, a medicine-taking-person initial column, a birth date column, a prescription date column, and a medicine administration start scheduled date. The research content page is provided with a medicine-taking-person background column, a combined medicine column, a column of findings during the drug-taking, a column of the presence of the adverse event, an adverse event column, a column of implementation of a clinical inspection, a clinical inspection column, a validity verifying column, and a column of a reason for discontinuance/dropout.

The medicine-taking-person background column is provided with columns for describing segment, height, weight, underlying disease, medical history, smoking history, hypersensitiveness identity, family history, menarche, menstrual disorder, pregnancy/childbirth history, last childbirth/miscarriage date, oral contraceptive taking history, name of the oral contraceptive within three months before taking the present drug, and taking period. The combined medicine column is provided with columns for describing the presence of a combined medicine, medicine name of each combined medicine and a reason for the medicine administration, administration route of each combined medicine, dose of each combined medicine per day, and an administration period of each combined medicine.

The column of the findings during the drug-taking is provided with columns for describing drug-taking start dates immediately before the start of the drug-taking and in first, second, and third periods, smoking, the present drug taking situations (taking situations in the periods, continuation to the next period), the presence of pregnancy, contraception other than the oral contraceptive, the presence of hormone withdrawal bleeding (menses), the incoming date, the number of retaining days, bleeding amount, menstrual pain, metrorrhagia, mastodynia, nausea, and vomitus. The column of the presence of the adverse event is provided with columns for describing the presence of the adverse event, the adverse event, and comment. The adverse event column is provided with columns for describing adverse event name, degree, appearance date, treatment (the present medicine, drug treatment, another therapy/treatment), exitus, causal relation with respect to the present medicine, factors (underlying disease/combined relation) other than the present medicine, and passing and therapy details.

Moreover, the column of the implementation of the clinical inspection is provided with a column for describing the presence of the implementation of the clinical inspection before/after the drug-taking. The clinical inspection value column is provided with columns for describing inspection items (weight, sitting position blood pressure, pulse rate) on each inspection date, various types of blood inspections (e.g., the number of platelets, etc.), blood biochemical inspection (e.g., total cholesterol, etc.), internal secretion (e.g., cortisol, etc.), urine inspection (e.g., protein, etc.), gynecological examination (e.g., uterine cancer cell check, etc.), physical examination, normal region of the facility of each inspection item, judgment of the abnormal fluctuation, and judgment of the abnormal fluctuation during tracking. The validity verifying column is provided with columns for describing validity verification, a factor for invalidity, comment and the like. The column of the reason for the discontinuance/dropout is provided with columns for describing the reason for the discontinuance/dropout, comment and the like.

As one example of the medicine research information collection form 20, the patient registration form for case report research concerning the marketed diabetes mellitus therapeutic drug is constituted of a registration form cover sheet and a research content page. The providing of the patient registration form itself, and a constitution of the form in a case where the patient registration form is generated have a wide variety depending on the pharmaceutical company which is a patient registration client. However, in this embodiment, the cover sheet is provided with a pharmaceutical company column (e.g., contract number, patient number), a title column, a facility name and location column, a facility name column, an in-facility department name column, a patient initial column, a carte number column, a doctor name and authentication column, and columns for describing a start date of the present medicine administration, a fasting blood sugar level within one month before the start of the present medicine administration, and a user history of the diabetes mellitus drug. The research content page is provided with a patient background column, a therapy column (the present medicine administration situation column), a therapy column (combined drug/combined therapy), a clinical course column, an effect judgment column, a clinical inspection value column, a clinical inspection value abnormal fluctuation column, an adverse event column, and a hypoglycemia column.

The patient background column is provided with columns for describing birth date, occupation, gender, pregnancy, height, weight, reason for use, segment, disease contraction period, family history, diabetic complication, another complication, medical history, and hypersensitiveness identity. The therapy column (the present medicine administration situation column) is provided with columns for describing the present drug dose, the number of administration times of the present medicine per day, a medicine administration period, and discontinuance/dropout. The therapy column (combined drug/combined therapy column) is provided with columns for describing the presence of the combined drug, medicine name, medicine administration path, dose per day, medicine administration period, reason for use, and the presence of the combined therapy and the therapy. The clinical course column is provided with columns for describing inspected values of each inspection item of four, eight, and twelve weeks after the medicine administration, and the inspected value of each inspection item during the discontinuance/dropout. The effect judgment column is provided with columns for describing general judgment, grounds for the judgment, and the comment.

The clinical inspection column is provided with columns for describing the facility normal region of each inspection item of hematological inspection, biochemical inspection, and urine inspection, values before the medicine administration, and inspected values during and after the medicine administration. The clinical inspection value abnormal fluctuation column is provided with columns for describing the abnormal fluctuation judged to be clinically significant, and the comment concerning the clinical inspection value abnormal fluctuation. The adverse event column is provided with columns for describing the presence of an adverse event, a type and an appearance date of the adverse event, seriousness, treatment (the present medicine administration, therapy), exitus, a causal relation with respect to the present medicine, another suspected combined medicine/combined therapy, and passing (describe appearance situation, symptom, treatment, etc. in time series) and comment from the appearance till the exitus. The hypoglycemia column is provided with columns for describing contents of the hypoglycemia, a appearance date, glucose level measurement during appearance, a drug-taking timing, meal contents immediately before the appearance, momentum immediately before the appearance, and passing (describe appearance situation, symptom, treatment, etc. in time series) and comment from the appearance till the exitus.

As presumed from the examples of these forms, as a minimum constitution, the items of the research information collection form include an item concerning a research particular in which at least a part of research information is handwritten or selected. Especially, in the medicine research information collecting form 20, the minimum item is at least a particular concerning the facility in which the medicine research is performed, a particular concerning a doctor who performs the medicine research, initials of a subject (in the case of the clinical trial) or a patient (in the case of the post-marketing research), or a particular concerning contents of the medicine research, or a combined particular of them, especially preferably a combination of all of these items. Above all, the item is at least a title concerning the medicine research, a particular concerning the medicine to be researched, a particular (e.g., facility name) concerning the facility in which the medicine research is performed, a particular (e.g., signature, stamp column of the doctor in charge of the clinical trial) concerning the doctor who performs the medicine research, initials (in addition to the subject and the patient, a drug-taking-person depending on the research contents in some case) of the subject (in the case of the clinical trial) or the patient (in the case of the post-marketing research), or a particular concerning the contents of the medicine research, or an combined item of them, especially preferably a combination of all these items. Preferable items of the contents of the medicine research include a particular concerning the subject or the patient, a particular concerning the inspection of the subject or the patient performed during the medicine administration, a particular concerning effects of the medicine administration to the subject or the patient, or a particular concerning an influence of the medicine administration on the subject or the patient, or a combination of them, especially preferably a combination of all of them.

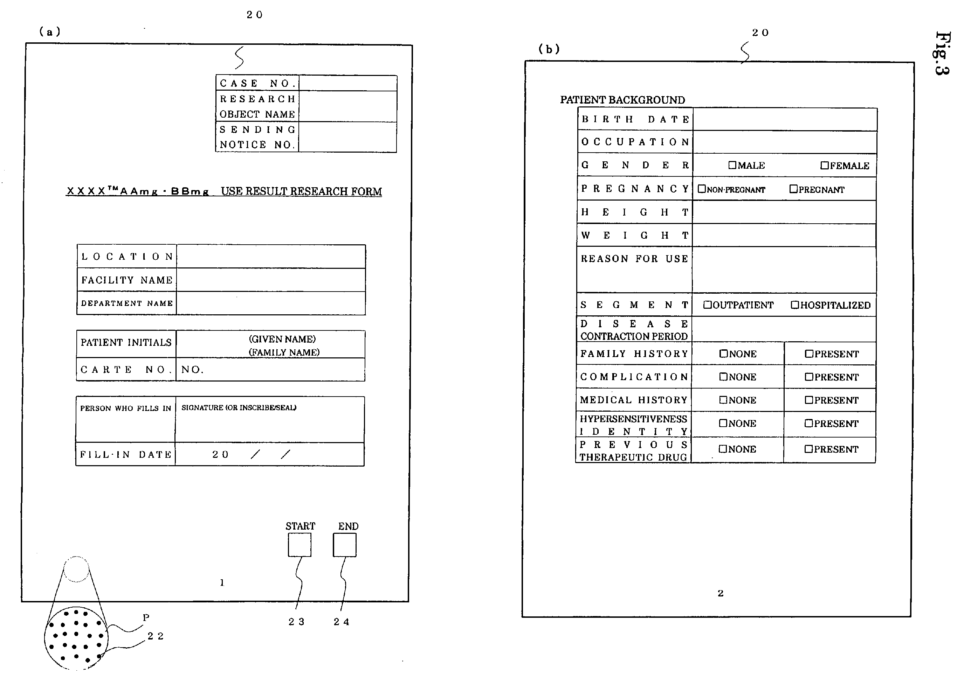

Here, as partially shown in FIGS. 1 and 3, the position encoding pattern P constituted by unevenly arranging fine raster points 22 is printed on the paper in each page of the medicine research information collection form 20. When the characters and the like are written in the medicine research information collection form 20 by use of the above-described electronic pen 10, this position encoding pattern P correctly specifies the position of the tip portion of the electronic pen 10 in the medicine research information collection form 20. A pattern constitution peculiarly differs with each page of the medicine research information collection form 20. That is, there is not another page on which the same pattern is printed in the medicine research information collection form 20. It is possible to specify the patient in the same facility by means of the position encoding pattern P peculiar to this medicine research information collection form 20. One raster point 22 as a constituting element of the position encoding pattern P is very fine. Therefore, the medicine research information collection form 20 of the present embodiment is visually recognized to be similar to usual paper or to be little colored.

Moreover, the characters, the frames and the like shown in each page of the medicine research information collection form 20 are printed from this position encoding pattern P. This position encoding pattern P is marked with, for example, printing ink or the like whose base is black carbon absorbing infrared light. Therefore, even when the characters and the like are printed on the position encoding pattern P, or written, the position encoding pattern P can be recognized without being interfered by the above-described printed characters or the like by use of a light emitting diode which emits the infrared ray and a sensor which is sensitive to the infrared light.

It is to be noted that the above-described example of marking the position encoding pattern P for the position encoding on the paper is a known technology described in, for example, Japanese Patent Application Laid-Open No. 2003-511761 and the like. Moreover, as the form marked with the position encoding pattern P on the paper as described above, for example, Anoto paper manufactured by Anoto Group AB is usable. A lower corner of the medicine research information collection form 20 is provided with a start check box 23 to be checked to start the fill-in, and an end check box 24 to be checked to complete the fill-in.

### (Constitution of primary database device)

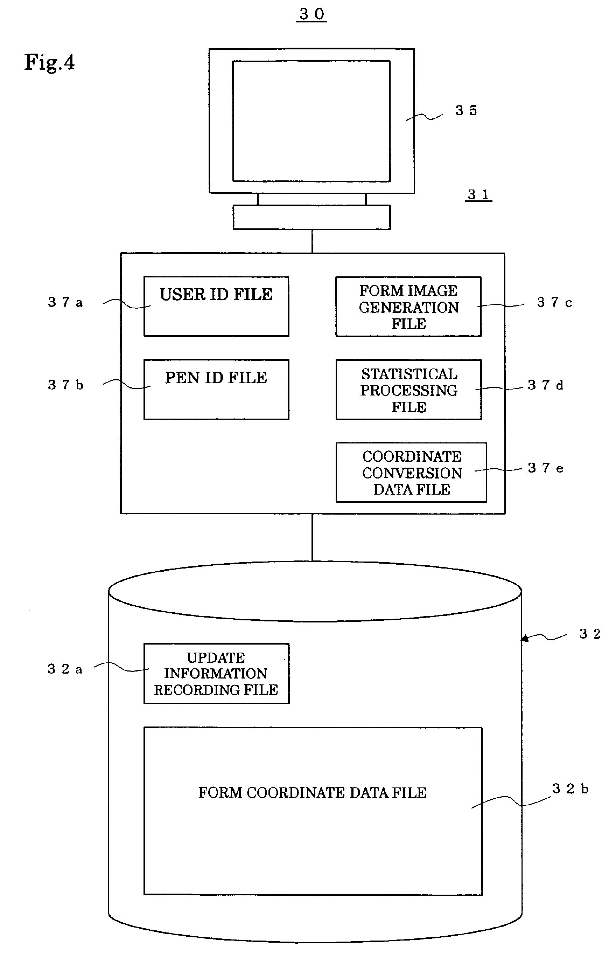

The primary database device 30 receives data successively transmitted from the electronic pen 10, accumulates the received data, and performs a confirmation operation for cleaning up this stored or a data update operation. As shown in FIG. 4, the primary database device 30 of the present embodiment is mainly constituted of a computer 31 and a primary database 32.

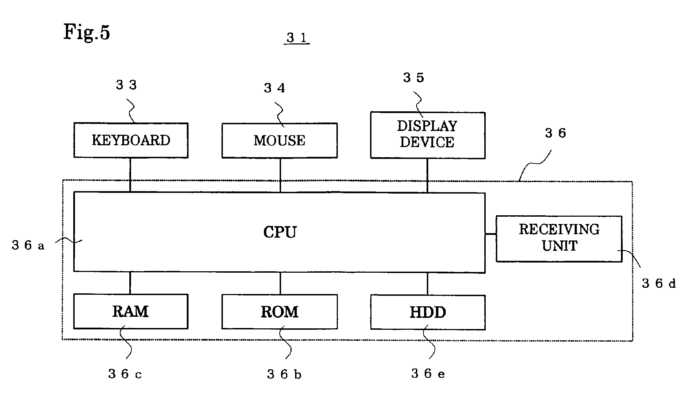

As shown in FIG. 5, the computer 31 is constituted of a keyboard 33, a mouse 34, a display device 35, and a computer main body 36. The computer main body 36 is provided with a CPU 36a, a ROM 36b, a RAM 36c, a receiving unit 36d, a hard disk 36e and the like. Various types of programs for operating the CPU 36a are stored in the ROM 36b, and the CPU 36a reads various types of programs stored in the ROM 36b to perform each processing described later. The RAM 36c is used as an operation area to store the data processed in the CPU 36a. The receiving unit 36d radio-communicates with an apparatus connected via Bluetooth (registered trademark) interface. The receiving unit 36d of the present embodiment receives various types of data transmitted from the transmission unit 16 built in the electronic pen 10.

In the hard disk 36e, as shown in FIG. 4, there are stored a user ID file 37a, a pen ID file 37b, a form image generation file 37c, a statistical processing file 37d, and a coordinate conversion data file 37e.

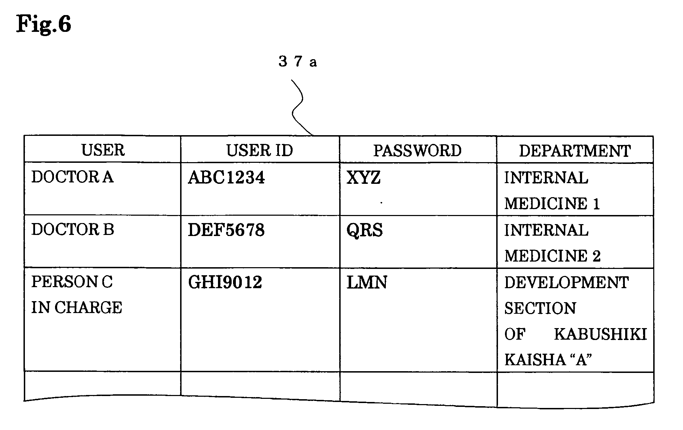

In the user ID file 37a, as shown in FIG. 6, there are registered names, user IDs, passwords, belonging departments and the like of doctors in charge of the clinical trial, pharmaceutical company persons in charge of the clinical trial and the like who are users. To utilize the primary database device 30, the user needs to input the user ID and the password in the display screen as described later. When the user ID and the password are input into the display screen, this input data is compared with the data stored in the user ID file 37a. When both data agree with each other, the user is permitted to use the primary database device 30.

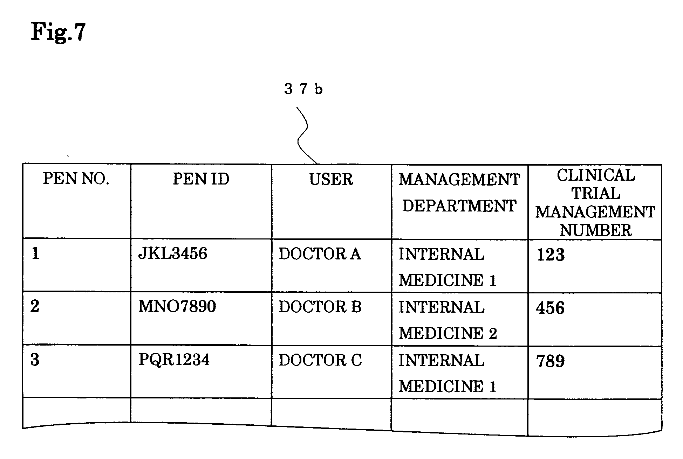

In the pen ID file 37b, as shown in FIG. 7, there are registered numbers of pens to be used, pen IDs, pen managers, management departments, clinical trial management numbers and the like. As to the electronic pen 10 for use in the clinical site, the electronic pen capable of transmitting the data to the primary database device 30 is limited to the pen registered in this pen ID. Accordingly, the pen ID can be acquired to specify the facility in which the electronic pen 10 is used. As described later, on receiving the pen ID signal transmitted from the electronic pen 10, the pen ID data included in this received signal is compared with the data stored in the pen ID file 37b. When both the data agree with each other, the data transmitted from the pen is permitted to be stored in the primary database device 30. The ID data transmitted from the pen can be received and analyzed to grasp the doctor in charge of the clinical trial, who uses the electronic pen 10, and the management number of the clinical trial being performed at present.

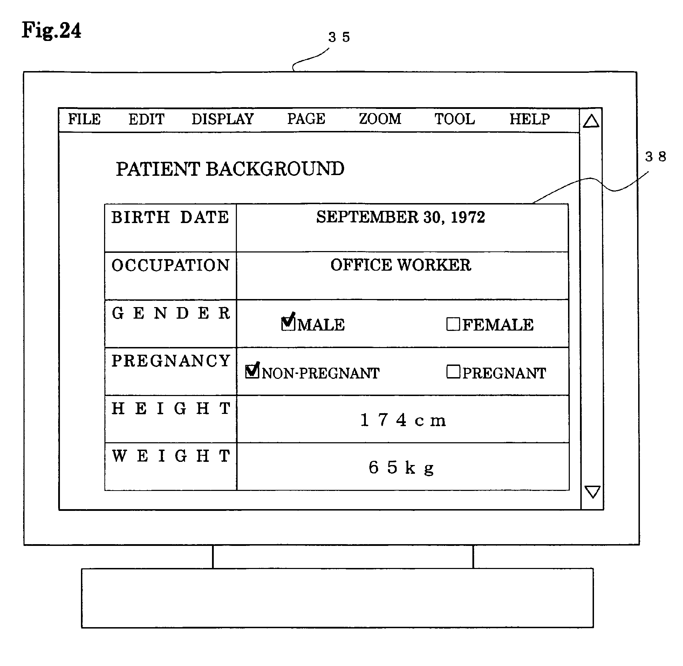

The form image generation file 37c allows the contents written in the medicine research information collection form 20 to be image-displayed as such in the screen of the display device 35 based on coordinate data stored in the primary database 32. In this form image generation file 37c, there are registered a plurality of image sheets 38 (see FIG. 24) corresponding to the pages of the medicine research information collection form 20. In the image sheets 38, as described later, there are displayed characters, codes, checkmarks and the like written in originals distributed to the clinical sites.

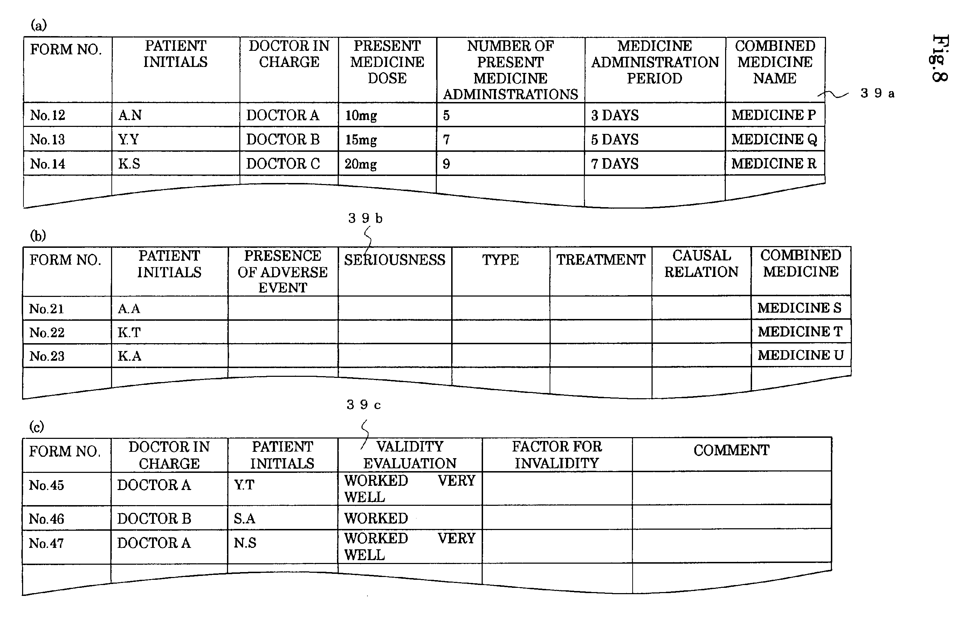

The statistical processing file 37d is stored in the primary database device 30, and information on the totaled medicine research information collection forms 20 is integrated for each item in the file, and displayed in the screen of the display device 35. In this statistical processing file 37d, as shown in FIG. 8, there are worksheets 39a, 39b, 39c ... having item columns corresponding to entry items disposed in the medicine research information collection form 20. In the worksheet 39a, patient initials, doctors in charge, present medicine doses, numbers of present medicine administration times, medicine administration periods, and combined medicine names are compiled every totaled forms. In the worksheet 39b, for example, patient initials, the presence of the adverse event, type, seriousness, treatment, causal relation, and combined drug are compiled every totaled forms. Furthermore, in the worksheet 39c, the doctors in charge, patient initials, validity evaluations, factors for invalidity, and comments are compiled every totaled forms.

Moreover, the worksheets 39a, 39b, 39c ... of the present embodiment can be freely edited so as to select the items to be displayed. As described later, the characters, codes, classifications and the like written in the originals distributed to the clinical sites are displayed in the cells of the worksheets 39a, 39b, 39c ... prepared by means of the statistical processing file 37d. A cell designating number is written in each cell of the worksheets 39a, 39b, 39c .... As such statistical processing file, for example, there is usable statistical software of SAS Co., Excel (registered trademark) of Microsoft Co. or the like.

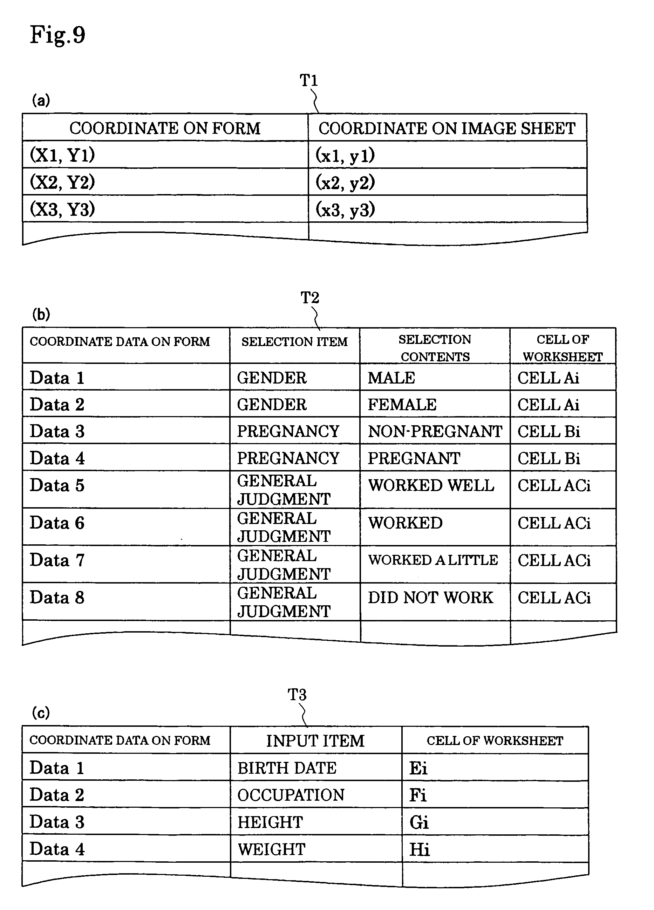

In the coordinate conversion data file 37e, a coordinate on the medicine research information collection form 20 is converted into that on the image sheet 38 (see FIG. 24). Selected contents provided with checkmarks are specified in selection columns disposed in the medicine research information collection form 20. The cells in the worksheets 39a, 39b, and 39c corresponding to the character input columns of the medicine research information collection form 20 are designated. As shown in FIG. 9, the coordinate conversion data file 37e is constituted of a plurality of data tables T1, T2, T3 ...

In the data table T1, each coordinate on the medicine research information collection form 20 is converted into that on the image sheet 38 (see FIG. 24). As shown in FIG. 9(a), the data table T1 is provided with columns of "the coordinate on the form" and "the coordinate on the image sheet". Coordinates {(X1, Y1), (X2, Y2), (X3, Y3) ...} registered in the column of "the coordinate on the form" indicate the coordinates on the medicine research information collection form 20, and coordinates {(x1, y1), (x2, y2), (x3, y3) ...} registered in the column of "the coordinate on the image sheet" indicate the coordinates on the image sheet 38. Moreover, in the data table T1, the coordinates {(X1, Y1), (X2, Y2), (X3, Y3) ...} registered in the column of "the coordinate on the form" correspond to the coordinates {(x1, y1), (x2, y2), (x3, y3) ...} registered in the column of "the coordinate on the image sheet". A relative position of "the coordinate on the form" in the medicine research information collection form 20 agrees with that of "the coordinate on the image sheet" in the image sheet 38.

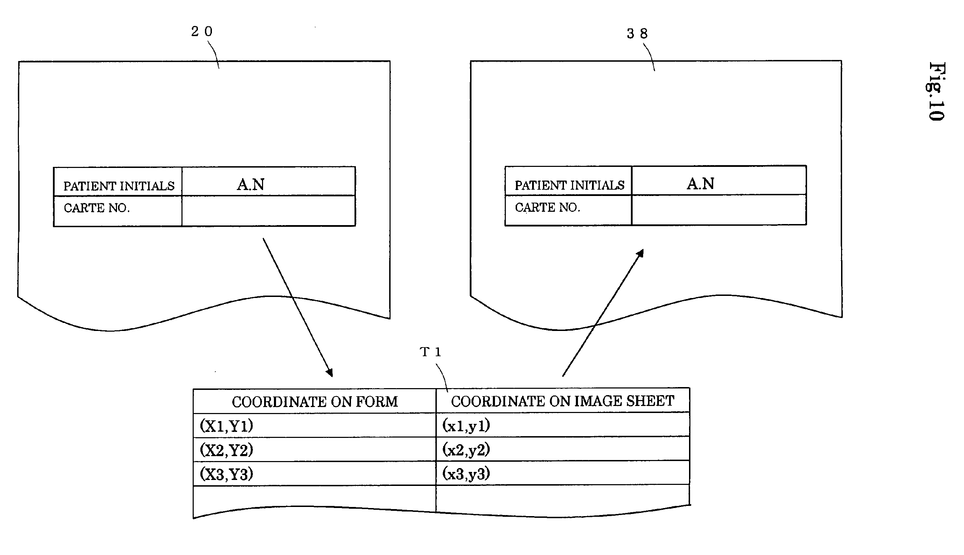

Moreover, the characters, diagrams, checkmarks and the like written in the medicine research information collection form 20 can be copied onto the image sheet 38 by use of this data table T1. For example, when the patient initial is written as "A. N" in the medicine research information collection form 20 as shown in FIG. 10, a series of coordinate data group at a time when the pen passes to write this "A. N" is stored in a form coordinate data file 32b (see FIG. 4). Moreover, the coordinates on the image sheet 38 corresponding to the series of coordinate data group are designated by use of the data table T1, and a point on the image sheet 38 corresponding to each designated coordinate is displayed to display the characters and the like written in the medicine research information collection form 20 as such on the image sheet 38.

In the data table T2, as shown in FIG. 9(b), the selected contents provided with checkmarks are specified from the selection columns disposed in the medicine research information collection form 20, and the cells in the worksheets corresponding to the items provided with the checkmarks are designated. The data table T2 is provided with columns of "the coordinate data on the form", "the selection item", "the selected contents", and "the cell of the worksheet". In the column of "the coordinate data on the form" of the data table T2, there is registered each coordinate data of a portion provided with a checkmark area on the medicine research information collection form. In the column of "the selection item" of the data table T2, there is registered each belonging item name of the checkmark area on the medicine research information collection form 20. In the column of "the selected contents" of the data table T2, the selected contents corresponding to the checkmark area on the medicine research information collection form are registered. In the column of "the cell of the worksheet" of the data table T2, there is registered the designated number of each cell in the worksheet.

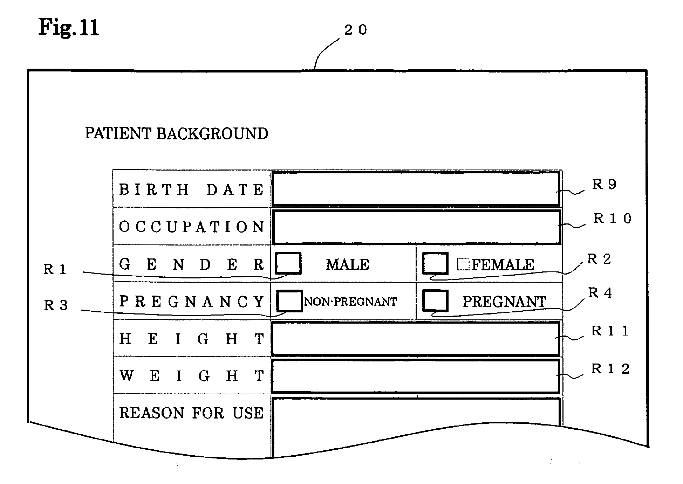

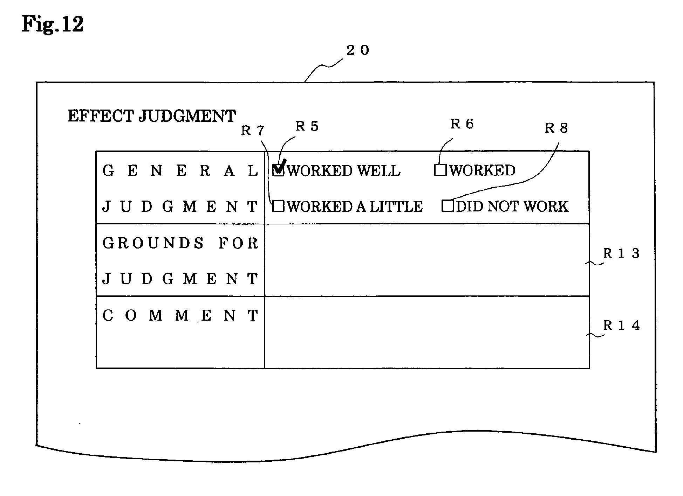

That is, with reference to the examples shown in FIGS. 11 and 12, in the column of "the coordinate data on the form" of the data table T2 shown in FIG. 9(b), there are registered coordinate data Data1, Data2, Data3 ... Data8 of portions provided with checkmark areas R1, R2, R3, R4, R5, R6, R7, and R8 on the medicine research information collection forms 20 shown in FIGS. 11 and 12. In the column of "the selection item" of data table T2 shown in FIG. 9, there are registered names of items to which the checkmark areas R1, R2, R3, R4, R5, R6, R7, and R8 belong on the medicine research information collection forms 20 shown in FIGS. 11 and 12, that is, "gender", "pregnancy", "general judgment" and the like.

Moreover, in the column of "the selected contents" of the data table T2 shown in FIG. 9(b), there are registered the selected contents represented by the checkmark areas R1, R2, R3, R4, R5, R6, R7, and R8 on the medicine research information collection forms 20 shown in FIGS. 11 and 12, that is, "male", "female", "non-pregnant", "pregnant", "worked well", "worked", "worked a little", "did not work" and the like. In the column of "the cell of the worksheet" of the data table T2, there are registered cell designating numbers {(Ai;A1, A2, A3 ...)} of the worksheet 39d shown in FIG. 13(a), which correspond to the "gender" registered in the column of the "selection item" of the data table T2. There are registered cell designating numbers {(Bi;B1, B2, B3 ...)} of the worksheet 39d shown in FIG. 13(a), which correspond to the "pregnancy" registered in the column of the "selection item" of the data table T2. There are registered cell designating numbers {(ACi;AC1, AC2, AC3 ...)} of the worksheet 39e shown in FIG. 13(b), which correspond to the "general judgment" of the data table T2. This cell designating number is counted up as i = 1, 2, 3 ... n every time the cell is designated.

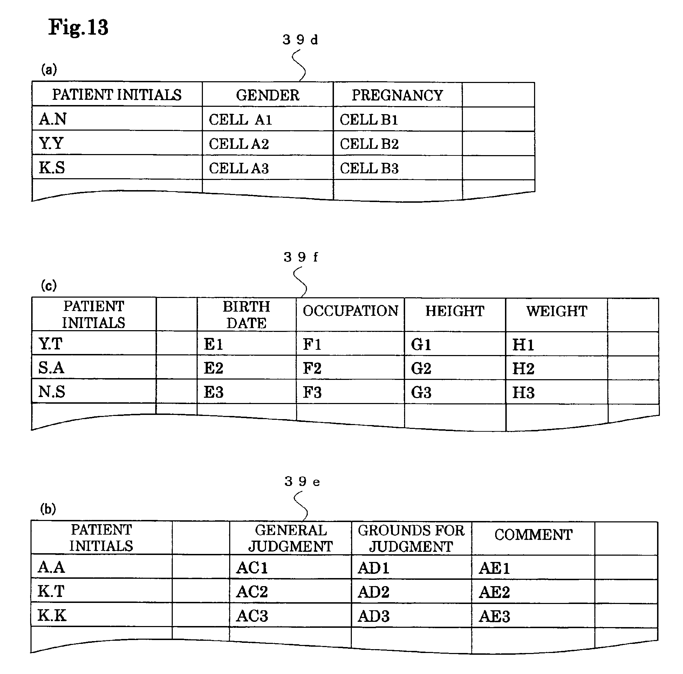

Furthermore, in a case where the checkmark is written in one of the selection items of each of the medicine research information collection forms 20 shown in FIGS. 11, 12 (e.g., in a case where the checkmark is attached to the checkmark area R1 shown in FIG. 11), agreement is judged between coordinate data (coordinate data detected by the pen) obtained by attaching this checkmark and coordinate data (e.g., Data1) registered in the column of "the coordinate data on the form" of the data table T2. In a case where they agree with each other, the contents of the selection item are specified in the column of the "selected contents" of the data table T2 (e.g. "male" is specified). In the data table T2, the "cell of the worksheet" corresponding to the "selected contents" specified in this manner is designated (e.g., the cell A1 is specified). The "selected contents" are registered in the worksheet cell designated in this manner (e.g., the "male" is registered in the cell A1 shown in FIG. 13(a)).

In the data table T3, as shown in FIG. 9(c), the cell of the worksheet corresponding to the character input column of the medicine research information collection form 20 is designated. As shown in FIG. 9(c), the data table T3 is provided with columns of "the coordinate data on the form", "the input item", and "the cell of the worksheet". In the column of "the coordinate data on the form" of the data table T3, there is registered each coordinate data of a portion provided with a character input area on the medicine research information collection form. In the column of "the input item" of the data table T3, there is registered each item name which belongs to the character input area on the medicine research information collection form. In the column of "the cell of the worksheet" of the data table T3, there is registered each designating number of the cell in the worksheet.

That is, with reference to the examples shown in FIGS. 11 and 12, in the column of "the coordinate data on the form" of the data table T3 shown in FIG. 9(c), there are registered coordinate data Data1, Data2, Data3 ... of portions provided with character input areas R9, R10, R11, R12, R13, and R14 on the medicine research information collection forms 20 shown in FIGS. 11 and 12. In the column of "the input item" of the data table T3 shown in FIG. 9(c), there are registered names of items which belongs to the character input areas R9, R10, R11, R12, R13, and R14 on the medicine research information collection forms 20 shown in FIGS. 11 and 12, that is, "birth date", "occupation", "height", "weight", "grounds for he judgment", "comment" and the like.

Moreover, in the column of "the cell of the worksheet" of the data table T3 shown in FIG. 9(c), there are registered cell designating numbers {(Ei;E1, E2, E3 ...)} of the worksheet 39f shown in FIG. 13(c), which correspond to the "birth date" registered in the column of the "input item" of the data table T3. There are registered cell designating numbers {(Fi;F1, F2, F3 ...)} of the worksheet 39f shown in FIG. 13(c), which correspond to the "occupation" registered in the column of the "input item" of the data table T3. There are registered cell designating numbers {(Gi;G1, G2, G3 ...)} of the worksheet 39f shown in FIG. 13(c), which correspond to the "height" of the data table T3. There are registered cell designating numbers {(Hi;H1, H2, H3 ...)} of the worksheet 39f shown in FIG. 13(c), which correspond to the "weight" of the data table T3. This cell designating number is counted up as i = 1, 2, 3 ... n every time the cell is designated.

Furthermore, in a case where the characters and the like are written in the column of the "input item" of the medicine research information collection forms 20 shown in FIGS. 11 and 12 (e.g., in a case where September 30, 1972 is written in the column of the birth date shown in FIG. 11), agreement is judged between coordinate data (coordinate data detected by the pen) obtained by writing the characters and the like and the coordinate data (e.g., Data1) registered in the column of "the coordinate data on the form" of the data table T3 shown in FIG. 13(c). In a case where they agree with each other, the "cell of the worksheet" corresponding to the "input item" is designated in the data table T3 (e.g., cell E1 is designated). Moreover, the characters and the like written in the column of the "input item" are registered as such in the worksheet cell designated in this manner (e.g., September 30, 1972 is registered in the cell E1 shown in FIG. 13(c)).

It is to be noted that character recognition software to recognize the characters (alphabets, Chinese characters, Japanese syllabary characters, etc.) may be installed beforehand in the computer 31 to recognize the characters using the software based on the coordinate data obtained by writing the characters in the medicine research information collection form 20. The recognized characters may be registered in the cell of the worksheet.

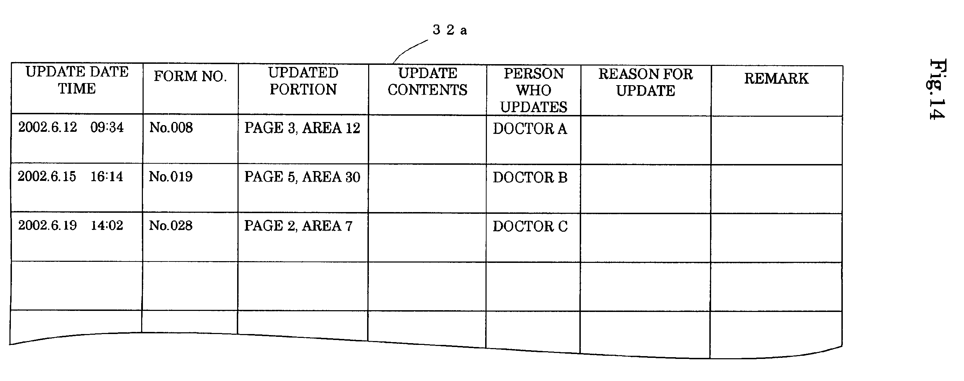

The primary database 32 stores the data transmitted from the electronic pen 10. In the primary database 32, as shown in FIG. 4, there are stored an update information recording file 32a and a form coordinate data file 32b. In the update information recording file 32a, as shown in FIG. 14, a date when input was performed, time, updated portion, update contents, a person who updated, reason for the update, remarks and the like are recorded in a case where the coordinate data stored in the form coordinate data file 32b is written and an update operation is performed to correct or delete the data. In the primary database device 30 of the present embodiment, when the above-described update operation is performed in an update mode, the updated contents are automatically recorded in the update information recording file 32a. It is to be noted that the reason for the update, remarks and the like may be appropriately input by a person in charge of the clinical trial by use of the keyboard 33 or the mouse 34.

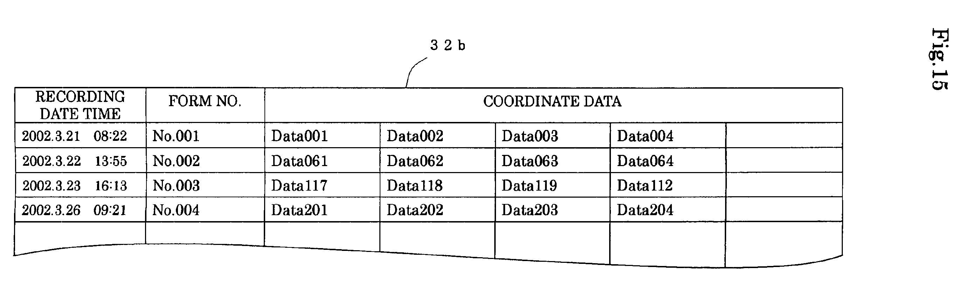

In the form coordinate data file 32b, as to the data successively transmitted from the electronic pen 10, there are stored a series of coordinate data concerning a movement track of the pen tip on the form in a case where the characters, diagrams, checkmarks and the like are written in the medicine research information collection form 20 by use of the electronic pen 10. The form coordinate data file 32b is constituted of a plurality of data tables. As partially shown in FIG. 15, recording date and time, form number, and coordinate data are recorded in these data tables in order of the transmission from the electronic pen 10.

### (Constitution of fixed database device)

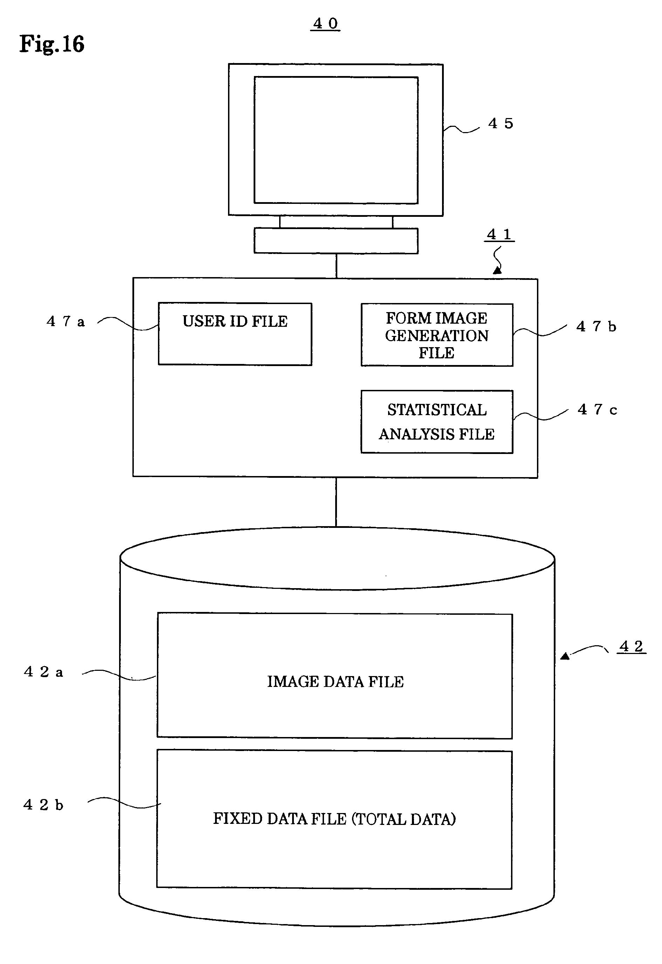

The fixed database device 40 stores the data fixed in the primary database device 30, and is usable by the pharmaceutical company person in charge of the clinical trial in performing statistical processing. As shown in FIG. 16, the fixed database device 40 of the present embodiment is mainly constituted of a computer 41 and a fixed database 42. Since hardware constitutions of the computer 41 and the fixed database 42 are the same as those of the above-described computer 31 and the primary database 32 of the primary database device 30, the description thereof is omitted. In a hard disk built in the computer 41, there are stored a user ID file 47a, a form image generation file 47b, and a statistical analysis file 47c.

The user ID file 47a is similar to the above-described user ID file 37a, and there are registered names, user IDs, passwords, belonging departments and the like of doctors in charge of the clinical trial (doctors in the post-marketing research), pharmaceutical company persons in charge of the clinical trial (persons in charge of the research in the post-marketing research) and the like who are users. To utilize the fixed database device 40, the user needs to input the user ID and the password in the display screen.

The form image generation file 47b allows the contents written in the medicine research information collection form 20 to be image-displayed as such in the screen of the display device 45 based on the coordinate data stored in the primary database 32. In an image data file 42a disposed in the fixed database 42, there are stored all of the image data of the totaled medicine research information collection form 20. This image data may be read to generate an image sheet by means of the form image generation file 47b, so that the sheet is displayed in a display device 45, or the medicine research information collection form 20 is printed via a printer (not shown).

The statistical analysis file 47c is for use in statistically processing or analyzing the data on the medicine research information collection form 20 fixed in a fixed data file 42b. In the fixed database 42, there are stored the image data file 42a and the fixed data file 42b. In the image data file 42a, there are stored all image data of the totaled medicine research information collection form 20. In the fixed data file 42b, all of the data of the totaled medicine research information collection form 20 are fixed and stored. It is possible to arrange the data of the form obtained in the clinical trial in time series or compile a causal relation between the inspection item and the medicine for use, dose, or the number of administration times for each patient who is a subject to be inspected.

It is to be noted that in the present embodiment, the fixed database device 40 is disposed separately from the primary database device 30, but, needless to say, the primary database device 30 and the fixed database device 40 may be constituted of a single database device.

### (Medicine research by medicine research information collection system)

Next, there will be described a method of performing medicine research by use of a medicine research information collection system S in one embodiment of the present invention. In the following description, as one example, there is shown an example of the medicine research in the clinical trial performed in a final stage of a new drug development process. The medicine research information collection form 20 is used in a case report form for use in the clinical trial. This medicine research information collection form 20 is distributed to the doctor in charge of the clinical trial so that one form can be prepared for one subject who is a clinical trial object. The medicine research information collection form 20 is filled in by use of the electronic pen 10 shown in FIG. 2.

First, the doctor in charge of the clinical trial performs medicine administration and inspection to the clinical trial object in accordance with a clinical trial implementation plan (step S101). Every time the medicine administration or the inspection is performed, implementation contents, symptom and the like are written in a carte (step S102). When an inspection result is obtained as an inspection form from a measurement device or the like, the inspection form is attached to the carte. Moreover, when the clinical trial is completed, predetermined particulars are transferred to the medicine research information collection form 20 based on the carte(step S103). When the fill-in is started, the start check box 23 of the medicine research information collection form 20 is checked.

The medicine research information collection form 20 is filled in by the doctor in charge of the clinical trial or by a nurse or the like under an instruction of the doctor in charge of the clinical trial. The doctor in charge of the clinical trial writes the location of the medical institution, the facility name, the department name, the initials of the subject as the clinical trial object, the carte number and the like in the predetermined columns of the medicine research information collection form 20. After the clinical test is actually performed, the doctor in charge of the clinical trial or the nurse transfers the information during the use of the investigational drug, the subject's states before and after the use of the investigational drug, and the clinical inspection results of blood, urine and the like, which are described in original data such as the carte and the inspection form, to the predetermined columns of the medicine research information collection form 20. Furthermore, the doctor in charge of the clinical trial directly writes, into the medicine research information collection form 20, items requiring medical judgment, safety evaluation and effect judgment of the investigational drug and the like, which are not described in the original data.

Moreover, when an adverse event or discontinuance/dropout occurs during the clinical trial, the contents are described in the medicine research information collection form 20. The adverse event refers to an unusual change of a certain symptom which has appeared in the clinical trial object, but a causal relation between the adverse event and the administered drug is unclear. When this adverse event appears, there are written, in the medicine research information collection form 20, predetermined particulars such as the presence of the adverse event, appearance date of the adverse event, type of adverse event, seriousness, treatment, the causal relation with respect to the administered drug, and combined drug.

Furthermore, when the discontinuance/dropout of the clinical trial occurs during the clinical trial, the contents are described in the medicine research information collection form 20. The discontinuance indicates that the clinical trial is discontinued, when a certain reason arises, and the doctor in charge of the clinical trial judges that the clinical trial cannot be continued any more. The dropout refers to a state in which the clinical trial cannot be continued because of the clinical trial object's bad condition. When this discontinuance/dropout occurs, the presence of the discontinuance/dropout, reason, cause and the like are written in the medicine research information collection form 20. When the contents are transferred from the carte to the medicine research information collection form 20, the pharmaceutical company person in charge of the clinical trial reviews the medicine research information collection form 20 (step S104).

Furthermore, after writing all the particulars into the medicine research information collection form 20, the doctor in charge of the clinical trial performs signature (step S105), thereby once completing the fill-in of the medicine research information collection form 20 in the clinical site. When the fill-in is completed, the end check box 24 of the medicine research information collection form 20 is checked. The medicine research information collection form 20 which has been filled in is collected by the clinical trial client.

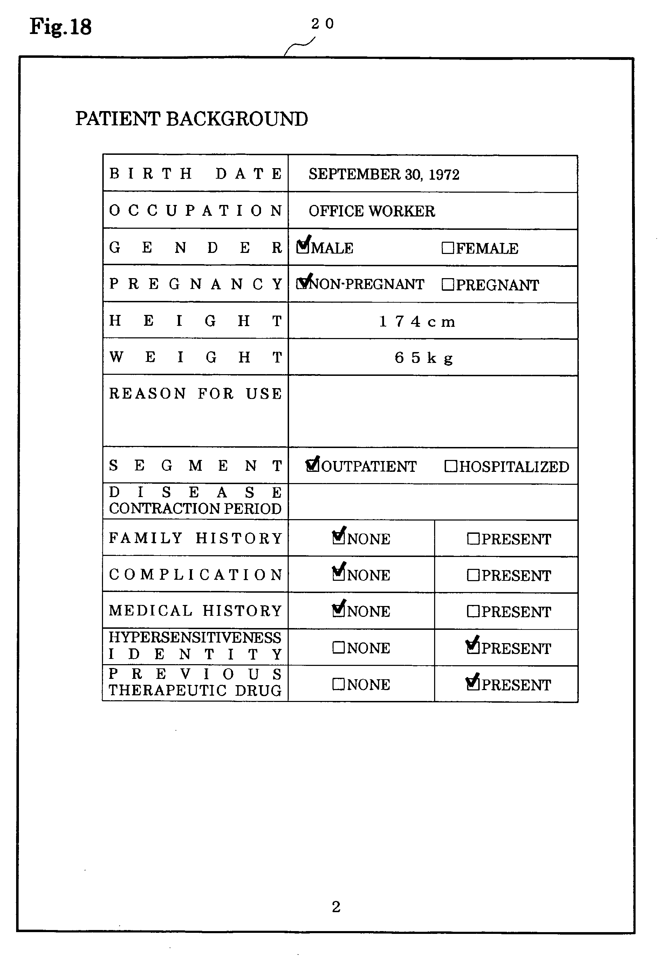

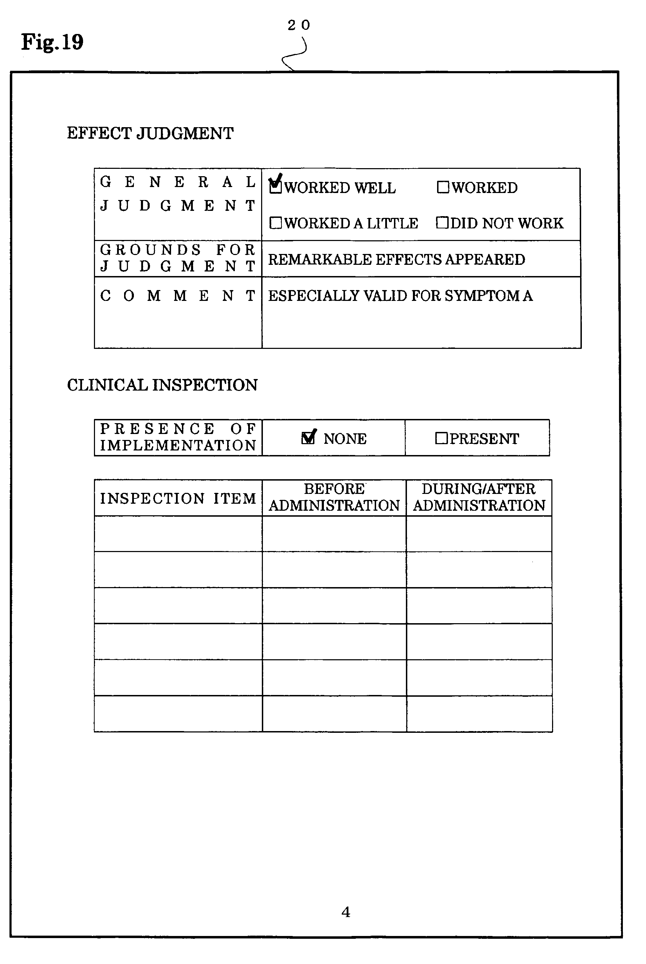

FIGS. 18 and 19 show one example in which predetermined particulars are written in the medicine research information collection form 20. FIG. 18 shows an example in which the use result research form is filled in. In columns of the patient background, birth date and the like are written, and checkmarks are attached to selection items. FIG. 19 shows an example in which the use result research form is filled in. The checkmark is attached to the selected item of the effect judgment, and grounds for judgment, comment and the like are written in a fill-in column. The characters, checkmarks and the like written in the medicine research information collection form 20 are made of ink fed from the ink unit 12 present in the electronic pen 10. It is to be noted that it has been described above in the embodiment that the input face of the medicine research information collection form 20 is paper, but the input face may be the screen of the display.

Next, the contents written in the medicine research information collection form 20 as described above are input into the primary database device 30 (step S106). When the contents written in the medicine research information collection form 20 are input into the primary database 32 of the primary database device 30, the information stored in the memory 15 of the electronic pen 10 may be transmitted to the primary database device 30, and stored.

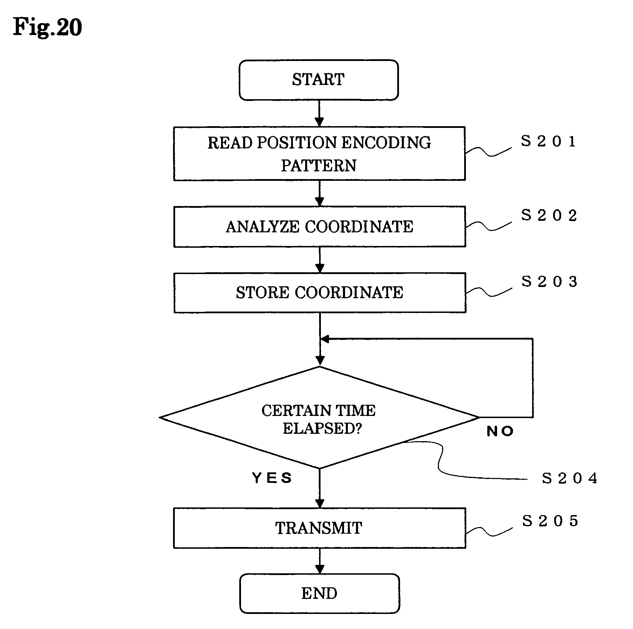

Here, FIG. 20 is a diagram showing a flow from a time when the contents written in the medicine research information collection form 20 by use of the electronic pen 10 are recorded until the contents are transmitted. First, in the electronic pen 10, the infrared ray is emitted from the light emitting diode 13a disposed in the camera 13 to the paper face of the medicine research information collection form 20. As described above, the position encoding pattern P including the raster points 22 arranged in one plane as described above is printed on the paper face, and a part of the infrared ray emitted from the light emitting diode 13a is absorbed by this position encoding pattern P. Moreover, the infrared ray is reflected by a portion of the paper face which is not provided with the position encoding pattern P, and this reflected light is received by the photosensitive area sensor 13b disposed in the pen camera. When the infrared ray reflected by the paper face is received in this manner, the photosensitive area sensor 13b can read the position encoding pattern P on the paper face on which the pen tip is positioned (step S201) .

Moreover, the read position encoding pattern is encoded in the electronic pen 10, and the encoded data is output to the processor unit 14, and stored. The processor unit 14 analyzes the coordinate based on the encoded data (step S202), and this coordinate is output to the memory 15. It is to be noted that in the camera 13, processing to emit the infrared light from the light emitting diode 13a and output the light signal received by the photosensitive area sensor 13b is performed, for example, 100 times per second. The data continuously output in this manner is successively transmitted in the electronic pen 10 and stored (step S203).

Moreover, in the processor unit 14, it is judged by a timer mechanism (not shown) whether or not a certain time has elapsed (step S204). In a case where it is judged that the certain time has elapsed (step S204: YES), the data stored in the memory 15 is automatically transmitted to the receiving unit 36d of the primary database device 30 via the transmission unit 16 without performing any special operation or the like by the doctor in charge of the clinical trial or the like (step S205). It is to be noted that the data stored in the memory 15 may be automatically transmitted to the receiving unit 36d of the primary database device 30 via the transmission unit 16 in response to the checking of the end check box 24. Moreover, a transmission button (not shown) may be attached to the electronic pen 10 beforehand so that the data stored in the memory is transmitted in response to pressing of this button. Furthermore, the data may be transmitted from the electronic pen 10 to the primary database device 30 via a portable terminal (cellular phone, PDA or the like) (not shown). The data may be transmitted from the electronic pen 10 to the primary database device 30 via internet.

In any case, in the present example, data indicating that the start check box 23 has been checked, data indicating that the end check box 24 has been checked, and data of the medicine research information written from a time when the start check box 23 is checked until the end check box 24 is checked are transmitted to the primary database device 30. In the present embodiment, since the data of the medicine research information from a time when the start check box 23 is checked until the end check box 24 is checked as described above is transmitted to the primary database device 30, the region of the data of the medicine research information written with the electronic pen 10 can be clarified. Accordingly, the data can be securely stored.

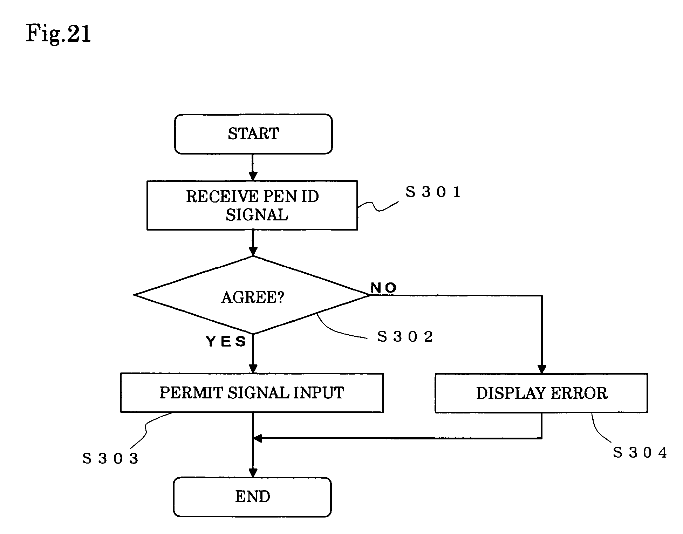

In the primary database device 30, the data transmitted from the electronic pen 10 is received by the receiving unit 36d as described above. In this case, a pen ID signal is transmitted from the electronic pen 10 together with the data stored in the memory 15. In the primary database device 30, this pen ID signal is received, and authentication processing of the electronic pen 10 is performed. FIG. 21 is a diagram showing a flow of the authentication processing of the electronic pen 10. First, when the pen ID signal is received in the primary database device 30 (step S301), it is judged whether or not pen ID data included in the pen ID signal agrees with the data stored in the pen ID file 37b (step S302). When both of the data agree with each other (step S302: YES), the data transmitted from the pen is permitted to be stored in the primary database device 30 (step S303).

On the other hand, when the pen ID data included in the pen ID signal does not agree with the data stored in the pen ID file 37b (step S302: NO), an error is displayed in the display device 35 (step S304). It is to be noted that the data may be transmitted from the electronic pen 10 to the primary database device 30 via a portable terminal (cellular phone, PDA or the like) (not shown). The data may be transmitted from the electronic pen 10 to the primary database device 30 via internet.

Moreover, in the primary database device 30, the received data is stored in the primary database 32. In the medicine research information collection system S of the present embodiment, the contents written in the medicine research information collection form 20 with the electronic pen 10 in this manner are computerized, and stored in the primary database 32. Therefore, unlike the conventional art, the contents written in the medicine research information collection form 20 do not have to be input into the primary database device 30 with the keyboard. Therefore, it is possible to rapidly improve an information collecting efficiency in the clinical trial. Since any input mistake is not generated during the input into the primary database device 30, the information obtained in the clinical trial can be correctly collected.

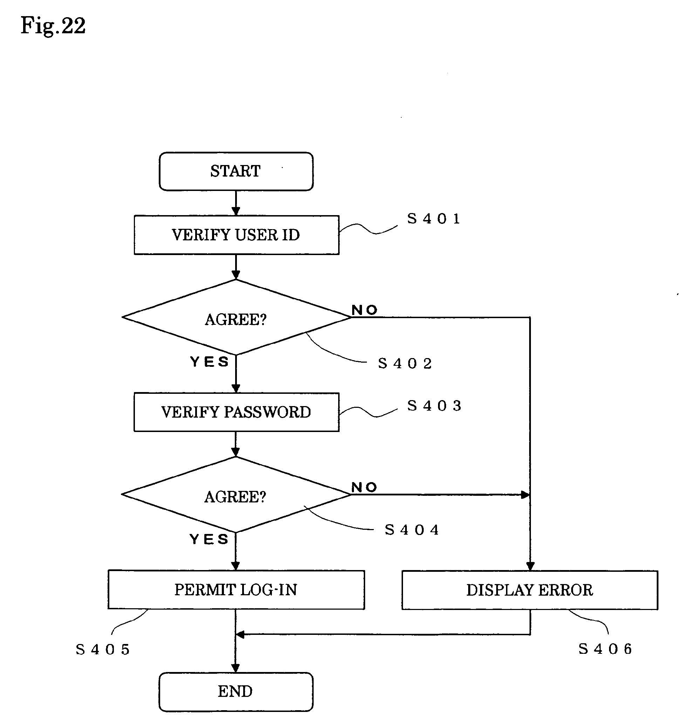

Furthermore, the contents of the primary database 32 stored as described above are cleaned up by the person in charge of the clinical trial (step S107). Here, the person in charge of the clinical trial needs to be authenticated as the user in the primary database device 30 in order to log in the primary database device 30. FIG. 22 is a diagram showing a flow of the user authentication in the primary database device 30. When the primary database device 30 is first started, for example, "please input your user ID and password" is displayed in the display screen of the display device 35, and there is displayed "the column to input the user ID and the password" in the vicinity. When the user inputs the user ID and the password into the above-described input column, the user ID is verified in the primary database device 30 (step S401).

Next, it is judged whether or not this input user ID agrees with the user ID predetermined in the user ID file 37a (step S402), and both of the IDs agree with each other (step S402: YES), the password is verified (step S403). Moreover, it is judged whether or not the input password agrees with the password predetermined in the user ID file 37a (step S404), and both of the passwords agree with each other (step S404: YES), the user is permitted to log in the primary database device 30 (step S405). On the other hand, when any of them is not verified (step S402: NO or step S404: NO), the error is not displayed in the display device 35 (step S406). Since the only specific user can be permitted to use the primary database device 30 in the present example, it is possible to prevent the leak of the medicine research information.

Moreover, when the person in charge of the clinical trial is permitted to log in the primary database device 30, the person reads and compares the contents described in the collected medicine research information collection form 20 and those of the primary database 32 stored as described above to confirm whether or not there is any mistake or unclear point in the contents stored in the primary database 32.

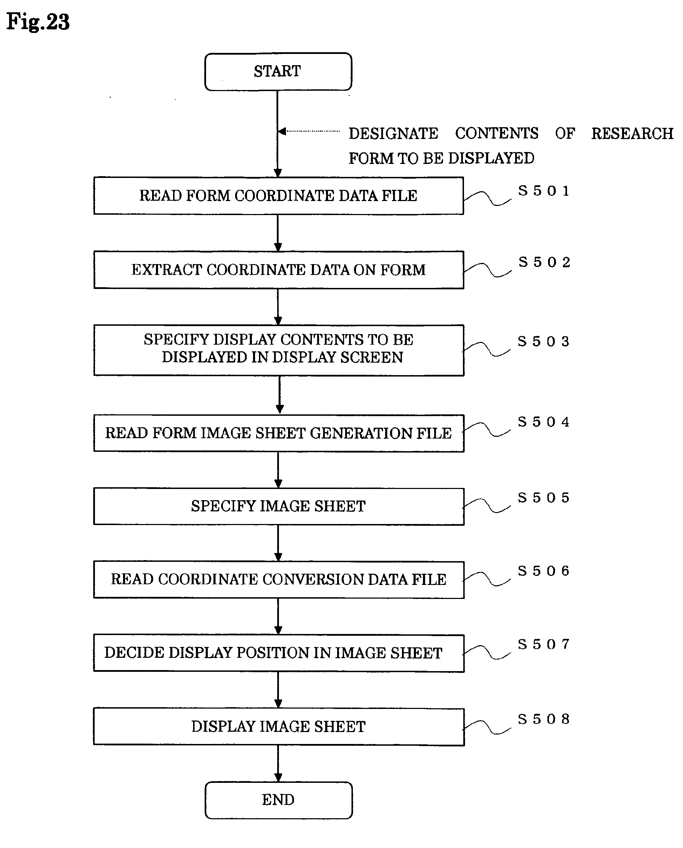

Here, in the primary database device 30 of the present embodiment, the information stored in the primary database 32 can be image-displayed in the display device 35. FIG. 23 is a diagram showing a flow of processing to image-display the medicine research information collection form 20. There will be described hereinafter an example to image-display the medicine research information collection form 20 (see FIGS. 18, 19) written in the steps S103 to S106.

First, to image-display the medicine research information collection form 20, the person in charge of the clinical trial designates the contents of the medicine research information collection form 20 to be displayed. For example, the clinical trial management number, date, form number and the like of the medicine research information collection form 20 to be image-displayed are input into the input columns (not shown) displayed in the display screen to designate the medicine research information collection form 20, and subsequently the page number and the like are designated. Accordingly, in the primary database device 30, the form coordinate data file 32b is read (step S501), and the coordinate data corresponding to the designation is extracted from the form coordinate data file 32b (step S502).

Moreover, the display contents displayed in the display screen are specified based on this coordinate data (step S503). Next, the form image generation file 37c is read (step S504), and the image sheet corresponding to the above-described designation is specified from the form image generation file 37c (step S505). Moreover, the coordinate conversion data file 37e is read (step S506), and there is determined the display position on the image sheet 38 in which the display contents indicated by the coordinate data extracted in the above-described step S502 are displayed based on the data table T1 (see FIG. 9) registered in this coordinate conversion data file 37e (step S507).

With reference to the shown example, in the patient background described in the medicine research information collection form 20 shown in FIG. 18, there is determined a relation between the coordinate data indicating "September 30, 1972" written in the input column of the birth date and the display position on the image sheet 38 based on the data table T1 shown in FIG. 9(a). Moreover, the designated display contents are displayed in the display position determined in this manner (step S508). That is, in the patient background described in the medicine research information collection form 20 shown in FIG. 18, "September 30, 1972" written in the input column of the birth date is displayed in the display column of the birth date on the image sheet 38 in the display device 35 as shown in FIG. 24. Similarly, in the patient background described in the medicine research information collection form 20 shown in FIG. 18, occupation, gender, pregnancy, height, weight and the like are displayed in display columns of the occupation, gender, pregnancy, height, weight and the like on the image sheet 38 in the display device 35 as shown in FIG. 24.

As described above, in the primary database device 30 of the present embodiment, the contents of the medicine research information collection form 20 written with the electronic pen 10 are displayed on the image sheet displayed in the display device 35. Therefore, the person in charge of the clinical trial can read and compare the contents described in the collected medicine research information collection form and those of the primary database 32 stored as described above.

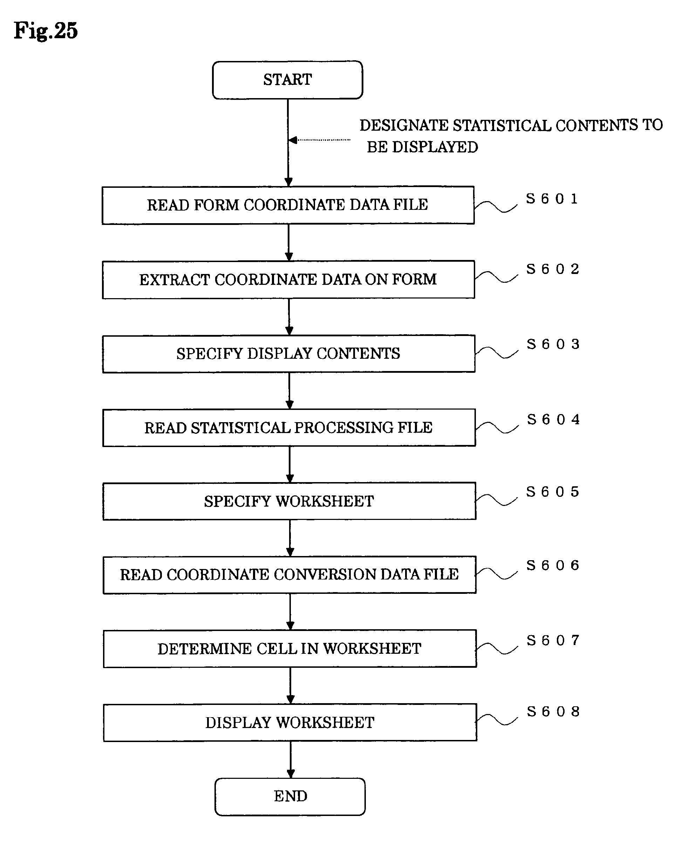

Moreover, in the primary database device 30 of the present embodiment, the information on the totaled medicine research information collection form 20 is compiled for each item, and this can be displayed in the screen of the display device 35. FIG. 25 is a diagram showing a flow of processing to compile the information on the totaled medicine research information collection form for each item and display the results in the screen of the display device. There will be described hereinafter an example in which statistical processing results are displayed with respect to the form (see FIGS. 18, 19) written in the steps S103 to S106.

First, the information on the totaled medicine research information collection form is compiled for each item, and the person in charge of the clinical trial designates the statistical contents to be displayed to display the results in the screen of the display device. For example, the statistical items and the like to be displayed are designated in the input column (not shown) displayed in the display screen. Accordingly, in the primary database device 30, the form coordinate data file 32b is read (step S601), and the coordinate data corresponding to the above-described designation is extracted from the form coordinate data file 32b (step S602). Moreover, the display contents displayed in the display screen are specified based on this coordinate data (step S603).

Next, the statistical processing file 37d is read (step S604), and the worksheet corresponding to the above-described designation is specified from the statistical processing file 37d (step S605). Moreover, the coordinate conversion data file 37e is read (step S606). Based on the data tables T2 and T3 (see FIG. 9) registered in this coordinate conversion data file 37e, the position of the cell of the worksheet is determined to display the display contents indicated by the coordinate data extracted in the step S602 (step S607).

With reference to the shown example, in the patient background described in the medicine research information collection form 20 shown in FIG. 18, there is determined a relation between the coordinate data indicating "male" provided with the checkmark in the selection item of the gender and the cell of the worksheet based on the data table T2. Moreover, the designated display contents are displayed in the cell determined in this manner (step S608). In the patient background described in the medicine research information collection form 20 shown in FIG. 18, the selected contents corresponding to the portion provided with the checkmark in the selection item of the gender are determined to be "male" by means of the data table T2, and the cell of the worksheet is further designated.

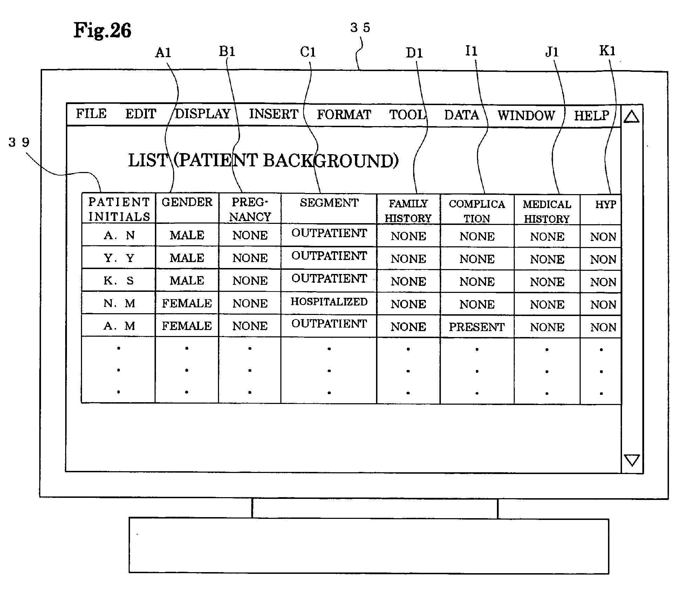

Accordingly, as shown in FIG. 26, characters "male" are written and displayed in the cell A1 of a worksheet 39. Similarly, in the patient background described in the medicine research information collection form 20 shown in FIG. 18, the selected contents corresponding to the portions provided with the checkmarks in the selection items of pregnancy, segment, family history, complication, medical history, hypersensitiveness identity, and previous therapeutic drug are determined, respectively, via the data table T2, and the cells of the worksheet 39 are designated. Accordingly, as shown in FIG. 26, characters "non-pregnant", "outpatient", "none", "none", "none", "none" ... are written and displayed in cells B1, C1, D1, I1, J1, K1 ... of the worksheet 39.

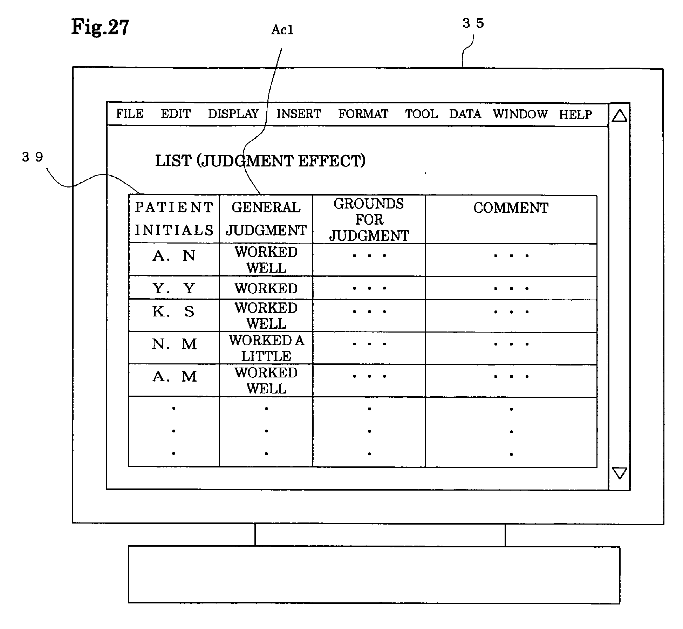

Moreover, in the effect judgment described in the medicine research information collection form 20 shown in FIG. 19, the selected contents corresponding to the portion provided with the checkmark in the selection item of the general judgment are determined to be "worked well" via the data table T2, and the cell of the worksheet is further designated. Accordingly, as shown in FIG. 27, characters "worked well" were written and displayed in the cell AC1 of the worksheet 39.

As described above, in the primary database device 30 of the present embodiment, the information on the totaled medicine research information collection form 20 is compiled for each item, and the result can be displayed in the screen of the display device 35. Therefore, the person in charge of the clinical trial can more efficiently read and compare the contents described in the collected medicine research information collection form 20 and those of the primary database 32 stored as described above.

Moreover, the person in charge of the clinical trial reads and compares the contents described in the collected medicine research information collection form 20 and those of the primary database 32 stored as described above to confirm whether or not the contents input into the primary database 32 have fill-in leak, fill-in mistake, cacography/lipography, protocol violation or the like. In a case where there is a mistake or an unclear point in the contents stored in the primary database 32, the doctor in charge of the clinical trial who has filled in the form is inquired (step S108).

In response to the inquiry, the doctor in charge of the clinical trial refers to the carte, the inspection form and the like again. In a case where there are the mistake, cacography/lipography, unclear description or the like in the particulars described in the medicine research information collection form 20, this effect is returned (step S109), and the medicine research information collection form 20 is corrected, and then signed (step S110). In this case, the correction or the signature is performed by use of the electronic pen 10 in the same manner as in the step S103.

It is to be noted that a checkbox for correcting the medicine research information collection form 20 is disposed beforehand, and this check box is checked to perform the correction. Accordingly, the primary database device 30 judges whether or not the medicine research information transmitted from the electronic pen 10 corrects that already stored in the primary database 32. When it is judged that the medicine research information transmitted from the electronic pen 10 corrects that already stored in the primary database 32, the medicine research information transmitted from the electronic pen 10 is stored as the corrected medicine research information in the primary database 32.

The corrected or signed contents are transmitted and stored in the primary database device 30 in this manner. In this case, the corrected contents are not overwritten, and are successively recorded in the update information recording file 32a. In the medicine research information collection system S of the present embodiment, a correction history can be prepared in this manner. Therefore, since all the input course can be traced, a fact that test data is corrected, changed, or deleted halfway and the contents of the data can be easily known later.

Moreover, in a case where it can be confirmed that there is not any more problem in the contents input into the primary database 32, the data input into the primary database 32 is fixed, and stored in the fixed database 42 (step S112). The data of the fixed database 42 fixed in this manner is statistically processed, finally complied as clinical result data, analysis report form or the like required for application for approval of manufacturing of the drug, and utilized in data for the approval of the manufacturing of the investigational drug (step S113). After completing the collection of the medicine research information in this manner, the medicine research information can be transferred to the fixed database device 40 to prevent information from being leaked or tampered.

As described above, according to the present embodiment, the medicine research information collection form 20 is constituted of the paper face having the raster points 22 which determine the coordinate position on the paper face, and the items for handwriting or selecting at least the information of medicine research are printed on the paper face. Therefore, it is possible to computerize the handwritten contents of the medicine research information collection form 20 based on the position encoding pattern P constituted of the raster points 22 by use of, for example, the electronic pen 10 capable of computerizing the handwritten contents. Furthermore, this electronic information can be accumulated to thereby more easily form the database. As described above, according to the present embodiment, since the contents written in the medicine research information collection form 20 can be directly organized into the database, it is possible to save the trouble of inputting, into the computer, the contents written in the medicine research information collection form 20 by the person in charge of the clinical trial or the like. The contents of the plurality of collected medicine research information collection forms 20 can be organized into the database with good efficiency.

Moreover, according to the present embodiment, as described above, the contents written in the medicine research information collection form 20 can be directly organized into the database. Therefore, without generating the input mistakes and the like in the database organizing stage, the contents of the medicine research information collection form 20 described by the doctor in charge of the clinical trial or the like can be correctly organized into the database,

### Industrial Applicability

A research information collection form and a research information collection method of the present invention are applicable to validity research and the like in the market before/after marketing in addition to the medicine research. In the present invention, without performing any manual operation for input from a signed original of a medicine research table into a database or software in a computer, a signed medicine research form can be electronized as such, and the present invention is also useful as a technology to develop formation of an electronic information original in the future.

### Brief Description of the Drawings

FIG. 1 is a diagram showing the whole constitution of a medicine research information collection system in an embodiment of the present invention;
FIG. 2 is a diagram showing a constitution of an electronic pen in the present embodiment;
FIG. 3 is a diagram showing a use result research form in the present embodiment;
FIG. 4 is a diagram showing a constitution of a primary database device in the present embodiment;
FIG. 5 is a diagram showing a constitution of a computer in the present embodiment;
FIG. 6 is a diagram showing a constitution of a user ID file in the present embodiment;
FIG. 7 is a diagram showing a constitution of a pen ID file in the present embodiment;
FIG. 8 is an explanatory view showing a constitution of a worksheet of a statistical processing file in the present embodiment;
FIG. 9 is an explanatory view showing a constitution of a data table of a coordinate conversion data file in the present embodiment;
FIG. 10 is an explanatory view of a function of the coordinate conversion data file in the present embodiment;
FIG. 11 is a first explanatory view showing arrangement positions of a checkmark area and a character input area in the present embodiment;
FIG. 12 is a second explanatory view showing the arrangement positions of the checkmark area and the character input area in the present embodiment;
FIG. 13 is an explanatory view showing a designated number of each cell in the worksheet of the present embodiment;
FIG. 14 is a diagram showing a constitution of an update information recording file in the present embodiment;
FIG. 15 is a diagram showing a constitution of a form coordinate data file in the present embodiment;
FIG. 16 is a diagram showing a constitution of a fixed database device in the present embodiment;

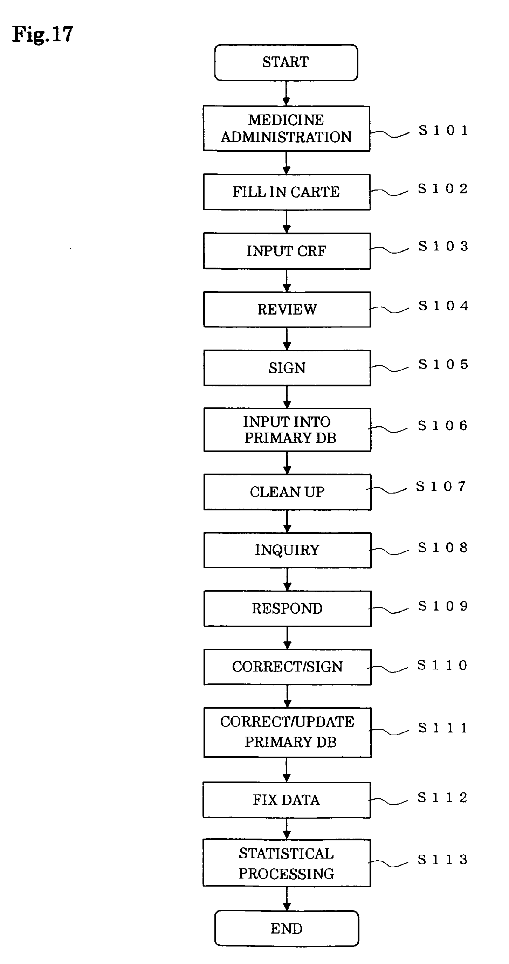

FIG. 17 is an explanatory view showing a flow of medicine research performed using the medicine research information collection system in the present embodiment;
FIG. 18 is a first explanatory view showing a fill-in example in a medicine research information collection form of the present embodiment;
FIG. 19 is a second explanatory view showing a fill-in example in the medicine research information collection form of the present embodiment;
FIG. 20 is a diagram showing a flow of signal processing in the electronic pen of the present embodiment;
FIG. 21 is a diagram showing a flow of authentication processing of pen ID in the primary database device of the present embodiment;
FIG. 22 is a diagram showing a flow of authentication processing of user ID in the primary database device of the present embodiment;
FIG. 23 is a diagram showing a flow of processing to display an image sheet in the present embodiment;
FIG. 24 is a diagram showing the image sheet displayed in a display device in the present embodiment;
FIG. 25 is a diagram showing a flow of processing to display a statistical processing result in the present embodiment;
FIG. 26 is a first explanatory view showing a worksheet displayed in the display device in the present embodiment; and
FIG. 27 is a second explanatory view showing the worksheet displayed in the display device in the present embodiment.

### Description of Reference Numerals

- 10: electronic pen
- 20: medicine research information collection form
- 22: raster point
- 30: primary database device
- 32: primary database
- 40: fixed database device
- 42: fixed database
- P: position encoding pattern
- S: medicine research information collection system

## Claims

1. A medicine research information collection system for collecting medicine research information, comprising:
a medicine research information collection form which has coordinate position deciding means for deciding a coordinate position on an input face and in which an item capable of handwriting or selecting at least the medicine research information is displayed in a field of the input face;
a handwriting device which is constituted to be capable of handwriting the medicine research information in the input face and which computerizes the handwritten medicine research information based on the coordinate position deciding means to transmit the information; and
a database device including a database which receives the medicine research information transmitted from the handwriting device and which accumulates the received medicine research information.

2. The medicine research information collection system according to claim 1, wherein the input face of the medicine research information collection form is provided with:
a start check box to be checked when the medicine research information starts to be written using the handwriting device; and
an end check box to be checked when the writing of the medicine research information is completed,
the handwriting device transmits an input start signal to the database device in response to the checking of the start check box, outputs an input end signal to the database device in response to the checking of the end check box, computerizes the medicine research information written in the input face of the medicine research information collection form from a time when the start check box is checked until the end check box is checked, and transmits the information to the database device, and
the database device starts the input of the medicine research information transmitted from the handwriting device based on the input start signal transmitted from the handwriting device, and completes the input of the medicine research information transmitted from the handwriting device based on the input end signal transmitted from the handwriting device.

3. The medicine research information collection system according to claim 1, wherein the database device comprises:
user identification information storage means for storing beforehand user identification information which identifies a user;
user identification information input means for the user to input the user's user identification information;
user identification information judgment means for judging whether or not the user identification information input by the user identification information input means agrees with that stored beforehand in the user identification information storage means; and
permission means for permitting the user to use the database device in a case where the user identification information input by the user identification information input means agrees with that stored beforehand in the user identification information storage means.

4. The medicine research information collection system according to claim 1, wherein the handwriting device is constituted to transmit identification information for identifying each handwriting device,
the database device comprises:
handwriting device identification information storage means for storing beforehand the identification information of each handwriting device;
identification information receiving means for receiving the identification information transmitted from the handwriting device; and
handwriting device identification means for judging whether or not the user identification information received by the identification information receiving means agrees with the identification information stored beforehand in the handwriting device identification information storage means to thereby identify the handwriting device which has transmitted the medicine research information.

5. The medicine research information collection system according to claim 1, wherein the database device comprises:
correction judgment means for judging whether or not the medicine research information transmitted from the handwriting device corrects the medicine research information already stored in the database; and
correction information storage means for storing the medicine research information transmitted from the handwriting device as corrected medicine research information in the database in a case where the correction judgment means judges that the medicine research information transmitted from the handwriting device corrects the medicine research information already stored in the database.

6. The medicine research information collection system according to claim 1, wherein the database device comprises: statistical processing means for statistically processing the medicine research information transmitted from the handwriting device.

7. The medicine research information collection system according to claim 1, wherein the database device comprises:
a primary database device which primarily stores the medicine research information transmitted from the handwriting device; and
a fixed database device which stores the medicine research information stored in the primary database device as fixed information.

8. A medicine research information collection form which has coordinate position deciding means for deciding a coordinate position on an input face and in which an item capable of handwriting or selecting at least the medicine research information is displayed in a field of the input face.

9. The medicine research information collection form according to claim 8, wherein the coordinate position deciding means is means for deciding the coordinate position of the handwriting device in the input face during the handwriting on the input face by use of the handwriting device.

10. The medicine research information collection form according to claim 8, wherein the item to be handwritten or selected is at least a particular concerning a facility in which medicine research is performed, a particular concerning a doctor who performs the medicine research, initials of a subject or a patient, or a particular concerning contents of the medicine research, or a combination of them, or the item is at least a title concerning the medicine research, a particular concerning the medicine to be subjected to the medicine research, the particular concerning the facility in which the medicine research is performed, the particular concerning the doctor who performs the medicine research, the initials of the subject or the patient, or the particular concerning the contents of the medicine research, or a combination of them.

11. The medicine research information collection form according to claim 10, wherein the contents of the medicine research are particulars concerning the subject or the patient, particulars concerning inspection of the subject or the patient, which is performed during medicine administration, particulars concerning effects of the medicine administration to the subject or the patient, or particulars concerning influences of the medicine administration on the subject or the patient, or a combination of them.

12. A medicine research information collection method for collecting medicine research information, the method comprising:
an input step of handwriting or selecting the medicine research information on an input face of a medicine research information collection form by use of a handwriting device;
a computerizing step of computerizing the medicine research information handwritten on the input face of the medicine research information collection form in the handwriting device;
a transmitting step of transmitting the medicine research information computerized in the computerizing step to a database device;
a receiving step of receiving the medicine research information transmitted from the handwriting device in the database device; and
an accumulating step of accumulating the medicine research information received in the receiving step in a database of the database device.

13. The medicine research information collection method according to claim 12, wherein when the information is handwritten on the input face of the medicine research information collection form by use of the handwriting device in the computerizing step, a coordinate position of the handwriting device in the input face is decided based on coordinate position deciding means disposed on the input face of the medicine research information collection form.

14. A medicine research information collection device for collecting medicine research information, comprising:
receiving means for receiving the medicine research information transmitted from a handwriting device capable of computerizing contents handwritten in a medicine research information collection form; and
a database which accumulates the medicine research information received by the receiving means.

15. A medicine research information collection program which allows a computer disposed in a medicine research information collection device for collecting medicine research information to execute:
a medicine research information receiving step of receiving the medicine research information transmitted from a handwriting device capable of computerizing contents handwritten in a medicine research information collection form; and
a medicine research information accumulating step of accumulating, in a database, the medicine research information received by the receiving step.

16. The medicine research information collection program according to claim 15, which allows the computer disposed in the medicine research information collection device to execute:
an input start step of starting input of the medicine research information transmitted from the handwriting device based on an input start signal transmitted from the handwriting device, when a start check box disposed in an input face of the medicine research information collection form is checked; and
an input end step of completing the input of the medicine research information transmitted from the handwriting device based on an input end signal transmitted from the handwriting device, when an end check box disposed in the input face of the medicine research information collection form is checked.

17. The medicine research information collection program according to claim 15, which allows the computer disposed in the medicine research information collection device to execute:
a user identification information storing step of storing beforehand user identification information which identifies a user;
a user identification information judging step of judging whether or not the user identification information input by user identification information input means disposed in the medicine research information collection device agrees with that stored beforehand in the user identification information storing step; and
a permitting step of permitting the user to use the medicine research information collection device in a case where it is judged that the user identification information input by the user identification information input means agrees with that stored beforehand in the user identification information storing step.

18. The medicine research information collection program according to claim 15, which allows the computer disposed in the medicine research information collection device to execute:
a handwriting device identification information storing step of storing beforehand identification information of each handwriting device;
an identification information receiving step of receiving the identification information transmitted from the handwriting device; and
a handwriting device identifying step of judging whether or not the user identification information received in the identification information receiving step agrees with the identification information stored beforehand in the handwriting device identification information storing step to thereby identify the handwriting device which has transmitted the medicine research information.

19. The medicine research information collection program according to claim 15, which allows the computer disposed in the medicine research information collection device to execute:
a correction judging step of judging whether or not the medicine research information transmitted from the handwriting device corrects the medicine research information already stored in a database; and
a correction information storing step of storing the medicine research information transmitted from the handwriting device as corrected medicine research information in the database in a case where correction judging step judges that the medicine research information transmitted from the handwriting device corrects the medicine research information already stored in the database.

20. The medicine research information collection program according to claim 15, which allows the computer disposed in the medicine research information collection device to execute:
a statistical processing step of statistically processing the medicine research information accumulated in the database.
